(19) Europäisches Patentamt
European Patent Office
Office européen des brevets

(11) Veröffentlichungsnummer : **0 094 668**
**B1**

(12) **EUROPÄISCHE PATENTSCHRIFT**

(45) Veröffentlichungstag der Patentschrift : **08.07.87**

(51) Int. Cl.[4] : **C 07 D487/04**, A 61 K 31/55 // (C07D487/04, 223:00, 209:00)

(21) Anmeldenummer : **83104815.2**

(22) Anmeldetag : **16.05.83**

(54) **Pyrrolo(3,4-d)(2)benzazepin-Derivate.**

(30) Priorität : **18.05.82 US 379400**
**28.06.82 US 393142**

(43) Veröffentlichungstag der Anmeldung : **23.11.83 Patentblatt 83/47**

(45) Bekanntmachung des Hinweises auf die Patenterteilung : **08.07.87 Patentblatt 87/28**

(84) Benannte Vertragsstaaten : **AT BE CH DE FR GB IT LI LU NL SE**

(56) Entgegenhaltungen :
**EP-A- 0 045 519**
**DE-A- 2 723 209**

(73) Patentinhaber : **F. HOFFMANN-LA ROCHE & CO. Aktiengesellschaft**

**CH-4002 Basel (CH)**

(72) Erfinder : **Fryer, Rodney Ian**
**5 Eton Drive**
**North Caldwell, N.J. (US)**
Erfinder : **Trybulski, Eugene J.**
**13 Homer Street**
**Parsippany, N.J. (US)**
Erfinder : **Walser, Armin**
**19 Crane Avenue**
**West Caldwell, N.J. (US)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 094 668 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Pyrrolo[3,4-d][2]-benzazepin-Derivate der allgemeinen Formel

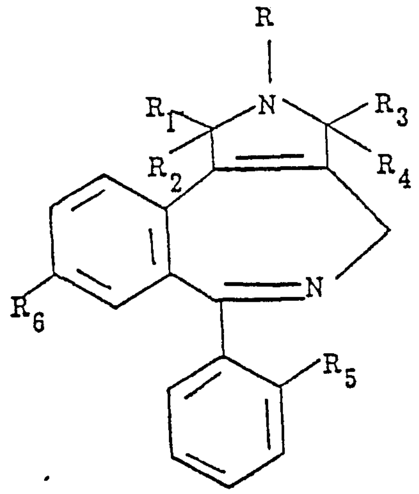

(I)

worin $R_1$ und $R_3$ Wasserstoff, $(C_1\text{-}C_7)$-Alkyl, Hydroxy, $(C_1\text{-}C_7)$-Alkoxy oder die Gruppe —OCOR', R' Wasserstoff, $(C_1\text{-}C_6)$-Alkyl oder Phenyl und $R_2$ und $R_4$ Wasserstoff oder $R_1$ und $R_2$ und/oder $R_3$ und $R_4$ zusammen Oxo-Gruppen bedeuten, mit der Massgabe, dass mindestens eine Oxo-Gruppe vorhanden ist, R Wasserstoff, $(C_1\text{-}C_7)$-Alkyl, Hydroxy-$(C_2\text{-}C_7)$-alkyl, Amino-$(C_2\text{-}C_7)$-alkyl oder mono- oder di-$(C_1\text{-}C_7)$-Alkylamino-$(C_2\text{-}C_7)$-alkyl oder die Gruppe -$(C_1\text{-}C_6)$-Alkyl-$COR^{21}$, $R^{21}$ Hydroxy, $(C_1\text{-}C_7)$-Alkoxy, Amino oder mono-oder di-$(C_1\text{-}C_7)$-Alkylamino, $R^5$ Halogen mit einer 35 nicht übersteigenden Ordnungszahl oder Wasserstoff und $R_6$ Halogen mit einer 35 nicht übersteigenden Ordnungszahl bedeuten, mit der Massgabe, dass wenn $R_1$ oder $R_3$ Hydroxy, $(C_1\text{-}C_7)$ Alkoxy oder —OCOR' bedeutet, R $(C_1\text{-}C_7)$-Alkyl oder Wasserstoff bedeutet, und die N-Oxide und die pharmazeutisch annehmbaren Salze davon.

Diese Verbindungen entfalten Wirkungen als Sedativa und Anxiolytika.

In EP-A2-45 519 sind Pyrrolo [3,4-d][2]benzazepine beschrieben, welche am Pyrrolring keine Oxogruppe(n) aufweisen.

Der Ausdruck « Halogen mit einer 35 nicht übersteigenden Ordnungszahl » bezeichnet Brom, Chlor oder Fluor, sofern nichts anderes angegeben ist.

Der Ausdruck « Alkyl » bezeichnet geradkettige oder verzweigte Kohlenwasserstoffreste, vorzugsweise $C_1\text{-}C_4$-Kohlenwasserstoffreste, wie Methyl, Aethyl, Propyl oder Isopropyl.

Reste der Formel —OCOR' sind z. B. Acetyl, Propionyl, Butyryl oder Benzoyl.

Der Ausdruck « pharmazeutisch annehmbare Salze » umfasst sowohl Salze mit anorganischen als auch Salze mit organischen, pharmazeutisch annehmbaren Säuren, wie Schwefelsäure, Salzsäure, Salpetersäure, Methansulfonsäure und p-Toluolsulfonsäure. Solche Salze können durch den Fachmann im Hinblick auf den Stand der Technik und die Natur der in ein Salz zu überführenden Verbindung leicht hergestellt werden.

Bevorzugte Verbindungen im Rahmen der vorliegenden Erfindungen sind diejenigen der Formel I, d. h. die Schiff'schen Basen. Weiterhin sind diejenigen Verbindungen der Formel I bevorzugt, worin $R_3$ und $R_4$ zusammen eine Oxo-Gruppe bedeuten. Weiterhin sind diejenigen Verbindungen der Formel I bevorzugt, worin $R_2$ Wasserstoff bedeutet. $R_1$ ist vorzugsweise Wasserstoff oder $(C_1\text{-}C_7)$-Alkyl, insbesondere Wasserstoff oder Methyl. Die bevorzugte Bedeutungsmöglichkeit von R ist Wasserstoff oder $(C_1\text{-}C_7)$-Alkyl, insbesondere Wasserstoff oder Methyl. $R_5$ ist vorzugsweise Halogen mit einer 35 nicht übersteigenden Ordnungszahl.

Aus dem vorstehenden folgt, dass eine besonders bevorzugte Gruppe von Verbindungen im Rahmen der vorliegenden Erfindung solche der Formel I sind, worin R und $R_1$ Wasserstoff oder $(C_1\text{-}C_7)$-Alkyl, insbesondere Methyl, $R_2$ Wasserstoff, $R_3$ und $R_4$ zusammen eine Oxo-Gruppe und $R_5$ Halogen mit einer 35 nicht übersteigenden Ordnungszahl bedeuten.

Bevorzugte Verbindungen sind :

8-Chlor-6-(2-fluorphenyl)-3,4-dihydropyrrolo[3,4-d]-[2]benzazepin-1(2H)-on,
8-Chlor-6-(2-chlorphenyl)-3,4-dihydropyrrolo[3,4-d]-[2]benzazepin-1(2H)-on,
8-Chlor-3,4-dihydro-6-phenylpyrrolo[3,4-d][2]benzazepin-1(2H)-on,
8-Chlor-6-(2-chlorphenyl)-3,4-dihydro-2-methyl-pyrrolo[3,4-d][2]benzazepin-1(2H)-on,
8-Chlor-6-(2-chlorphenyl)-1,4-dihydro-1-hydroxypyrrolo[3,4-d][2]benzazepin-3(2H)-on,
8-Chlor-6-(2-chlorphenyl)-3,4-dihydro-3-hydroxypyrrolo[3,4-d][2]benzazepin-1(2H)-on,
8-Chlor-6-(2-chlorphenyl)pyrrolo[3,4-d][2]benzazepin-1,3-(2H,4H)-dion,

8-Chlor-6-(2-chlorphenyl)-1,4-dihydropyrrolo[3,4-d]-[2]benzazepin-3(2H)-on-5-oxid,
8-Chlor-6-(2-chlorphenyl)-3,4-dihydropyrrolo[3,4-d]-[2]benzazepin-1(2H)-on-5-oxid,
8-Chlor-6-(2-chlorphenyl)-1-äthyl-1,4-dihydropyrrolo-[3,4-d][2]benzazepin-3(2H)-on,
8-Chlor-6-(2-chlorphenyl)-1,4-dihydro-1-methyl-3-oxo-2H-pyrrolo[3,4-d][2]benzazepin-2-essigsäure-methylester und
8-Chlor-6-(2-chlorphenyl)-1,4-dihydro-1-methyl-3-oxo-2H-pyrrolo[3,4-d][2]benzazepin-2-acetamid.

Besonders bevorzugte Verbindungen sind :

8-Chlor-6-(2-fluorphenyl)-1,4-dihydropyrrolo[3,4-d]-[2]benzazepin-3(2H)-on,
8-Chlor-1,4-dihydro-6-phenylpyrrolo[3,4-d][2]benzazepin-3(2H)-on,
8-Chlor-6-(2-chlorphenyl)-1,4-dihydro-1,2-dimethylpyrrolo[3,4-d][2]benzazepin-3(2H)-on und
8-Chlor-6-(2-chlorphenyl)-1,4-dihydro-2-(2-hydroxyäthyl)-1-methylpyrrolo[3,4-d][2]benzazepin-3(2H)-on.

Die am meisten bevorzugten Verbindungen sind :

8-Chlor-6-(2-chlorphenyl)-1,4-dihydropyrrolo[3,4-d]-[2]benzazepin-3(2H)-on,
8-Chlor-6-(2-chlorphenyl)-1,4-dihydro-1-methylpyrrolo[3,4-d][2]benzazepin-3(2H)-on und
8-Chlor-6-(2-chlorphenyl)-1,4-dihydro-2-methylpyrrolo[3,4-d][2]benzazepin-3(2H)-on.

Die Verbindungen der Formel I, die N-Oxide und die pharmazeutisch annehmbaren Salze davon können erfindungsgemäss dadurch hergestellt werden, dass man

a) zur Herstellung von Verbindungen der obigen Formel I, worin $R_1$ oder $R_3$ Wasserstoff oder ($C_1$-$C_7$)-Alkyl bedeutet und die übrigen Substituente die obige Bedeutung besitzen, und von N-Oxiden davon, eine Verbindung der allgemeinen Formel

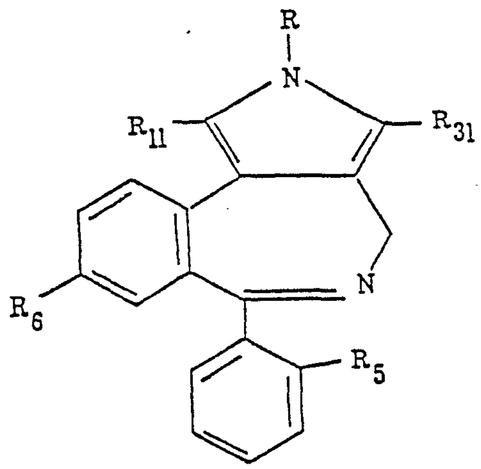

(II)

worin eines von $R_{11}$ und $R_{31}$ Wasserstoff und das andere Wasserstoff oder ($C_1$-$C_7$)-Alkyl bedeutet und R, $R_5$ und $R_6$ obige Bedeutung besitzen, mit einer Persäure oxidiert, oder

b) zur Herstellung von Verbindungen der obigen Formel I, worin $R_1$ oder $R_3$ Hydroxy bedeutet und die übrigen Substituenten die obige Bedeutung besitzen oder worin $R_1$ und $R_2$ und $R_3$ und $R_4$ jeweils zusammen Oxogruppen bedeuten und die übrigen Symbole die obige Bedeutung besitzen, eine Verbindung der Formel I, worin $R_1$ oder $R_3$ Wasserstoff bedeutet und die übrigen Substituenten die obige Bedeutung besitzen, oder eine Verbindung der obigen Formel II, worin $R_{11}$ und $R_{31}$ Wasserstoff bedeuten, mit einem Blei-(IV)-carboxylat in Gegenwart einer starken Säure oxidiert oder

c) zur Herstellung von Verbindungen der obigen Formel I, worin $R_1$ oder $R_3$ Acetoxy bedeutet und die übrigen Substituenten die obige Bedeutung besitzen, eine Verbindung der Formel I, worin $R_1$ oder $R_3$ Wasserstoff bedeutet und die übrigen Substituenten die obige Bedeutung besitzen, oder eine Verbindung der obigen Formel II, worin $R_{11}$ und $R_{31}$ Wasserstoff bedeuten, mit Blei-tetraacetat in Essigsäure behandelt oder

d) zur Herstellung von Verbindungen der obigen Formel I, worin $R_1$ oder $R_3$ —OCOR′ bedeutet und die übrigen Substituenten die obige Bedeutung besitzen, eine Verbindung der Formel I, worin $R_1$ oder $R_3$ Hydroxy bedeutet und die übrigen Substituenten die obige Bedeutung besitzen, in Gegenwart einer Base mit einem Carbonsäureanhydrid behandelt oder

e) zur Herstellung von Verbindungen der obigen Formel I, worin $R_1$ oder $R_3$ ($C_1$-$C_7$)-Alkoxy bedeutet und die übrigen Substituenten die obige Bedeutung besitzen, eine Verbindung der Formel I, worin $R_1$ oder $R_3$ —OCOR′ oder Hydroxy bedeutet und die übrigen Substituenten die obige Bedeutung

besitzen, oder eine Verbindung der Formel

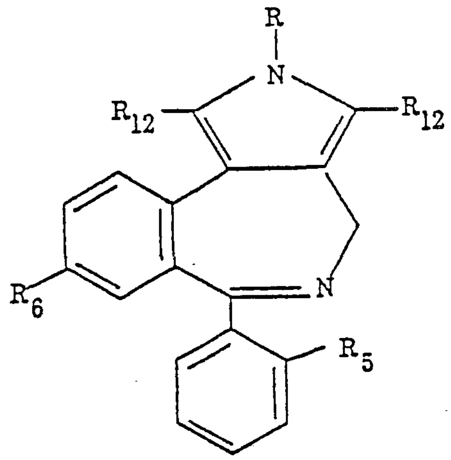

(III)

worin $R_{12}$ Acetoxy bedeutet und R, $R_5$ und $R_6$ die obige Bedeutung besitzen, in Gegenwart einer starken Säure mit einem ($C_1$-$C_7$)-Alkanol behandelt oder

f) zur Herstellung von Verbindungen der obigen Formel I, worin $R_1$ oder $R_3$ Wasserstoff oder ($C_1$-$C_7$)-Alkyl bedeutet und die übrigen Substituenten die obige Bedeutung besitzen, ein N-Oxid der allgemeinen Formel

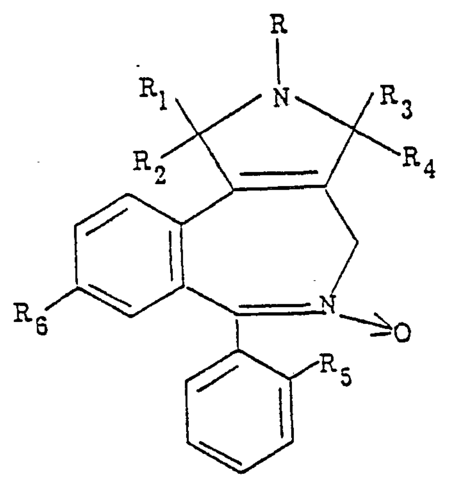

(IA)

worin R, $R_1$, $R_2$, $R_3$, $R_4$, $R_5$ und $R_6$ obige Bedeutung besitzen, desoxidiert und erwünschtenfalls

g) eine Verbindung der Formel I in ein pharmazeutisch annehmbares Salz oder ein pharmazeutisch annehmbares Salz einer Verbindung der Formel I in ein anderes pharmazeutisch annehmbares Salz überführt.

Die Oxidation einer Verbindung der Formel II mit einer Persäure gemäss Aspekt a) des erfindungsgemässen Verfahrens wird in an sich bekannter Weise durchgeführt. Geeignete Persäuren für diesen Oxidationsschritt sind Persäuren, wie m-Chlorperbenzoesäure, Pertrifluoressigsäure und dergleichen. In Abhängigkeit von den verwendeten Reaktionsbedingungen erhält man entweder die entsprechenden Verbindungen der Formel I oder die N-Oxide davon. Um die Verbindungen der Formel I zu erhalten, erfolgt die Reaktion zweckmässigerweise in einer niederen Carbonsäure, wie Essigsäure, Trifluoressigsäure, Propionsäure und dergleichen, oder in einer Mischung einer solchen Carbonsäure mit einem geeigneten inerten Lösungsmittel, wie ein chlorierter Kohlenwasserstoff, z. B. Methylenchlorid, ein aromatischer Kohlenwasserstoff, z. B. Benzol, Toluol und dergleichen, in Gegenwart einer starken Mineralsäure, wie Schwefelsäure und dergleichen. Falls ein entsprechendes N-Oxid erwünscht ist, erfolgt die Reaktion zweckmässigerweise in einem geeigneten inerten Lösungsmittel, wie ein chlorierter Kohlenwasserstoff, z. B. Methylenchlorid, oder ein aromatischer Kohlenwasserstoff, z. B. Benzol, Toluol und dergleichen, in Abwesenheit einer starken Mineralsäure. Die Oxidation einer Verbindung der Formel II wird vorzugsweise bei einer Temperatur zwischen etwa 0 °C und etwa Raumtemperatur durchgeführt. Es ist zu beachten, dass eine Mischung der 1-Oxo- und 3-Oxo-Verbindungen erhalten wird, wenn im Ausgangsmaterial der Formel II $R_{11}$ und $R_{31}$ beide Wasserstoff bedeuten. Falls die Oxidation unter stark sauren Bedingungen durchgeführt wird, überwiegt die 3-Oxo-Verbindung, wogegen die 1-Oxo-Verbindung das überwiegende Produkt ist, wenn die Reaktion in Abwesenheit einer starken Mineralsäure

durchgeführt wird. Die erhaltene Mischung kann nach gebräuchlichen Methoden, wie fraktionierte Kristallisation und Chromatographie, aufgetrennt werden.

Die Oxidation gemäss Aspekt b) des erfindungsgemässen Verfahrens wird ebenfalls nach an sich bekannten Methoden durchgeführt. In dieser Oxidation verwendet man zweckmässigerweise Blei-(IV)-carboxylate, wie Bleitetraacetat oder Blei-(IV)-trifluoracetat, in einem inerten Lösungsmittel, wie chlorierte Kohlenwasserstoffe, z. B. Methylenchlorid oder Chloroform, und dergleichen, in Gegenwart einer starken Säure, wie Trifluoressigsäure und dergleichen. Die Reaktion wird zweckmässigerweise in einem Temperaturbereich von etwa 0 °C bis etwa Raumtemperatur durchgeführt. Im Fall einer Verbindung der Formel II, worin $R_{11}$ und $R_{31}$ Wasserstoff bedeuten, erhält man eine Mischung der 1-Hydroxy-3-oxo-, der 3-Hydroxy-1-oxo- und der 1,3-Dioxo-Verbindungen. Die so erhaltene Mischung kann durch gebräuchliche chromatographische Massnahmen aufgetrennt werden.

Auch die Reaktion gemäss Aspekt c) des erfindungsgemässen Verfahrens wird nach an sich bekannten Methoden durchgeführt. So kann man das Ausgangsmaterial mit Bleitetraacetat in Essigsäure reagieren lassen, vorzugsweise bei etwa Raumtemperatur. Falls eine Verbindung der Formel II, worin $R_{11}$ und $R_{31}$ Wasserstoff bedeuten, als Ausgangsmaterial verwendet wird, erhält man ein Gemisch der 1-Acetoxy-3-oxo- und der 3-Acetoxy-1-oxo-Verbindungen und einer 1,3-Bis-acetoxy-Verbindung der Formel III. Die erhaltene Mischung kann durch gebräuchliche chromatographische Massnahmen aufgetrennt werden.

Die Reaktion einer Verbindung der Formel I, worin $R_1$ oder $R_3$ Hydroxy bedeutet, mit einem Carbonsäureanhydrid gemäss Aspekt d) des erfindungsgemässen Verfahrens erfolgt ebenfalls in an sich bekannter Weise, vorzugsweise in Gegenwart einer Base, wie Pyridin, Dimethylaminopyridin, N-Methylpiperidin und dergleichen. Inerte Lösungsmittel, welche bei dieser Reaktion verwendet werden können, sind chlorierte Kohlenwasserstoffe, wie Methylenchlorid, Aether, wie Tetrahydrofuran, und dergleichen. Die Reaktion wird zweckmässigerweise bei einer Temperatur im Bereiche von etwa 0 °C bis etwa Raumtemperatur durchgeführt.

Die Verätherung gemäss Aspekt e) des erfindungsgemässen Verfahrens erfolgt in an sich bekannter Weise, zweckmässigerweise durch Umsetzung einer Verbindung der Formel I, worin $R_1$ oder $R_3$ —OCOR′, vorzugsweise Acetoxy, bedeutet, oder einer Verbindung der Formel III mit einem Ueberschuss eines niederen Alkanols, enthaltend eine katalytische Menge einer starken Säure, wie Methansulfonsäure und dergleichen. Diese Reaktion wird vorzugsweise bei einer Temperatur zwischen etwa 0 °C und etwa Raumtemperatur durchgeführt.

Die Desoxidation gemäss Aspekt f) des erfindungsgemässen Verfahrens wird ebenfalls nach an sich bekannten Methoden durchgeführt, zweckmässigerweise durch Behandlung einer Verbindung der Formel IA mit einem Desoxidierungsmittel, wie Phosphortrichlorid oder Triphenylphosphin, in einem inerten Lösungsmittel, wie chlorierte Kohlenwasserstoffe, z. B. Methylenchlorid, aromatische Kohlenwasserstoffe, z. B. Toluol, Aether, z. B. Tetrahydrofuran, und dergleichen, bei einer Temperatur zwischen etwa Raumtemperatur und der Rückflusstemperatur des Lösungsmittels.

Die Ausgangsprodukte der Formel II sind bekannt oder können in einer zur Herstellung der bekannten Verbindungen analogen Weise hergestellt werden. Die nachfolgenden Reaktionsschemata illustrieren die Herstellung dieser Verbindungen der Formel II.

(Siehe Schema I Seite 6 f.)

worin $R_{13}$ Wasserstoff oder $(C_1$-$C_7)$-Alkyl bedeutet und $R_5$ und $R_6$ obige Bedeutung besitzen.

III → IV

Die Verbindung der Formel III kann in Gegenwart einer Base, wie Natriumhydrid, unter Verwendung einer Mischung von Dimethylsulfoxid und einem Aether, wie Diäthyläther, Dioxan oder Tetrahydrofuran, als Lösungsmittel mit einem α-Tosyl-alkylisocyanid umgesetzt werden. Die Reaktionstemperatur kann zwischen etwa 0 °C und etwa 40 °C liegen, wobei Raumtemperatur bevorzugt ist. Die oben erwähnten α-Tosyl-alkylisocyanide können gemäss van Leusen et al., Tetrahedron Letters, 3 487 (1975) hergestellt werden.

IV → IIa

Die Verbindung der Formel IV kann bei Drucken von etwa Atmosphärendruck bis 5 Atmosphären in Gegenwart eines Uebergangsmetallkatalysators, wie Raney-Nickel, unter Verwendung von Eisessig als Lösungsmittel mit Wasserstoff umgesetzt werden. Die Reaktionstemperatur liegt zweckmässigerweise bei etwa Raumtemperatur.

Das zuerst gebildete offenkettige Amin wird nicht isoliert, sondern cyclisiert spontan zum Produkt IIa.

Schema I

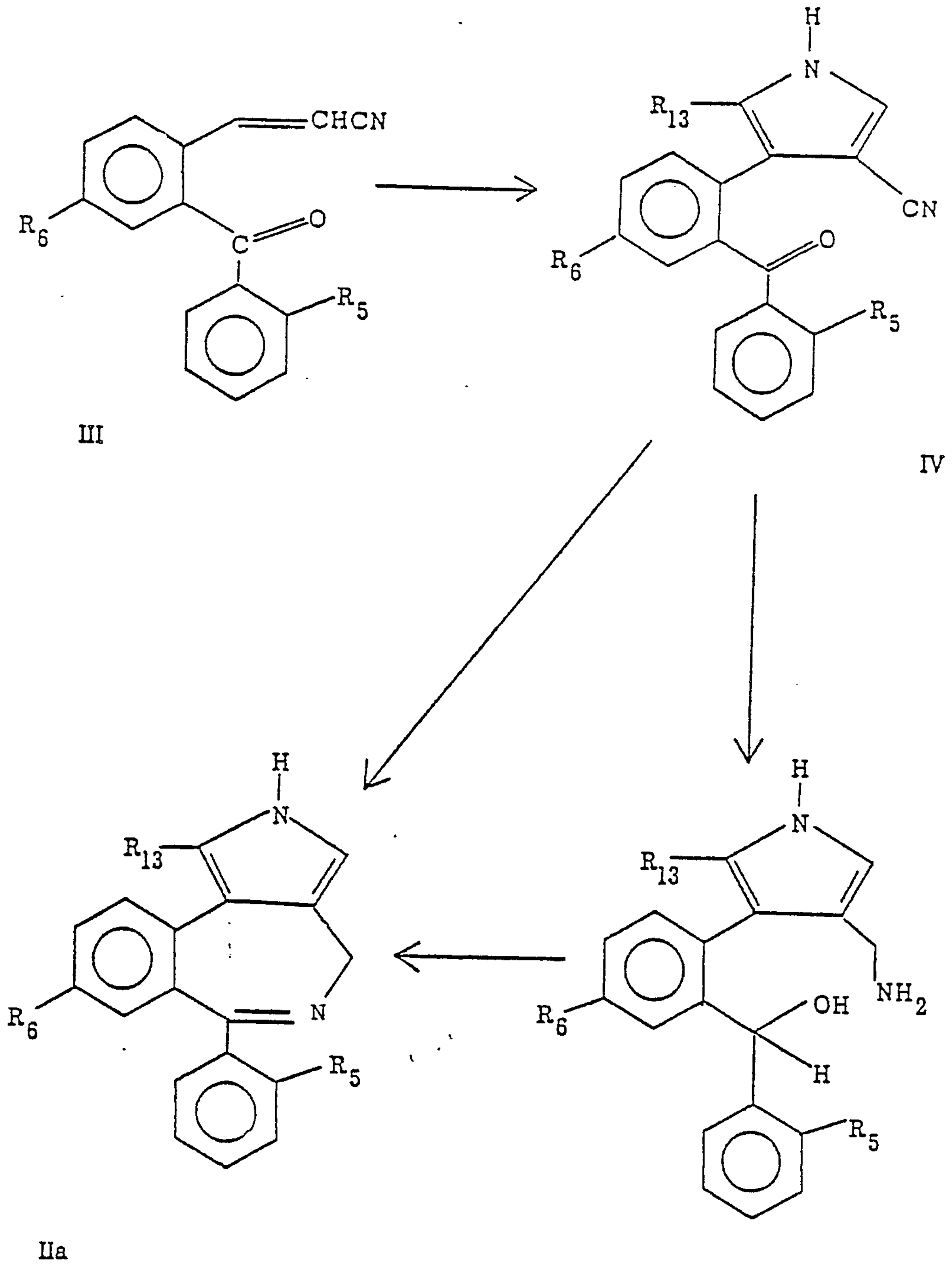

III

IV

IIa

V

IV → V

Die Verbindung der Formel IV kann in einem ätherischen Lösungsmittel, wie Tetrahydrofuran, mit einem Metallhydrid-Reduktionsmittel, wie Lithiumaluminiumhydrid, umgesetzt werden. Die Reaktionstemperatur kann in einem Bereich von etwa — 20 °C bis etwa Raumtemperatur liegen, wobei etwa 0 °C bevorzugt ist.

V → IIa

Die Verbindung der Formel V kann in einem ätherischen Lösungsmittel, wie Tetrahydrofuran, oder in einem anderen geeigneten Lösungsmittel, wie Toluol, mit Mangandioxid umgesetzt werden. Das entstehende Amin cyclisiert spontan zu Produkt IIa. Die Reaktionstemperatur kann zwischen etwa Raumtemperatur und dem Siedepunkt des Lösungsmittels liegen, wobei etwa 40 °C bevorzugt ist.

Schema II

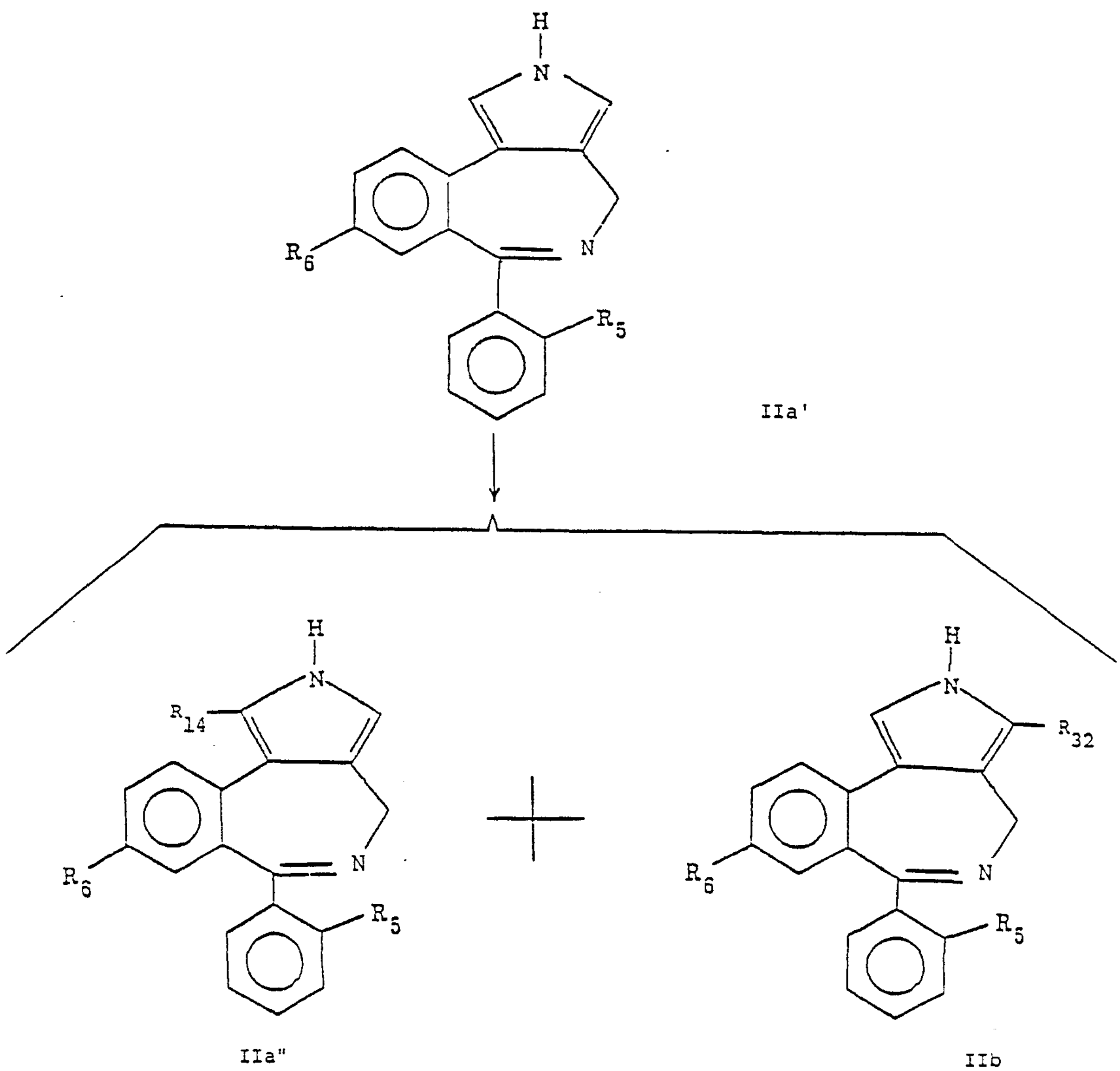

IIa'

IIa"

IIb

worin $R_{14}$ und $R_{32}$ $(C_1$-$C_7)$-Alkyl bedeuten und $R_5$ und $R_6$ die obige Bedeutung besitzen.

IIa' → IIa" + IIb

Die Verbindung der Formel IIa' kann zwischen etwa — 80 °C und etwa 0 °C, vorzugsweise bei etwa — 20 °C, mit einem Aequivalent einer starken Base, wie Lithiumdiisopropylamid, umgesetzt werden. Das

entstehende Anion wird mit dem gewünschten Alkylierungsmittel, wie einem niederen Alkyl-halogenid oder -sulfonat, behandelt. Man erhält eine Mischung der Isomeren der Formeln IIa" und IIb, welche durch gebräuchliche säulenchromatographische Massnahmen aufgetrennt werden kann.

Schema III

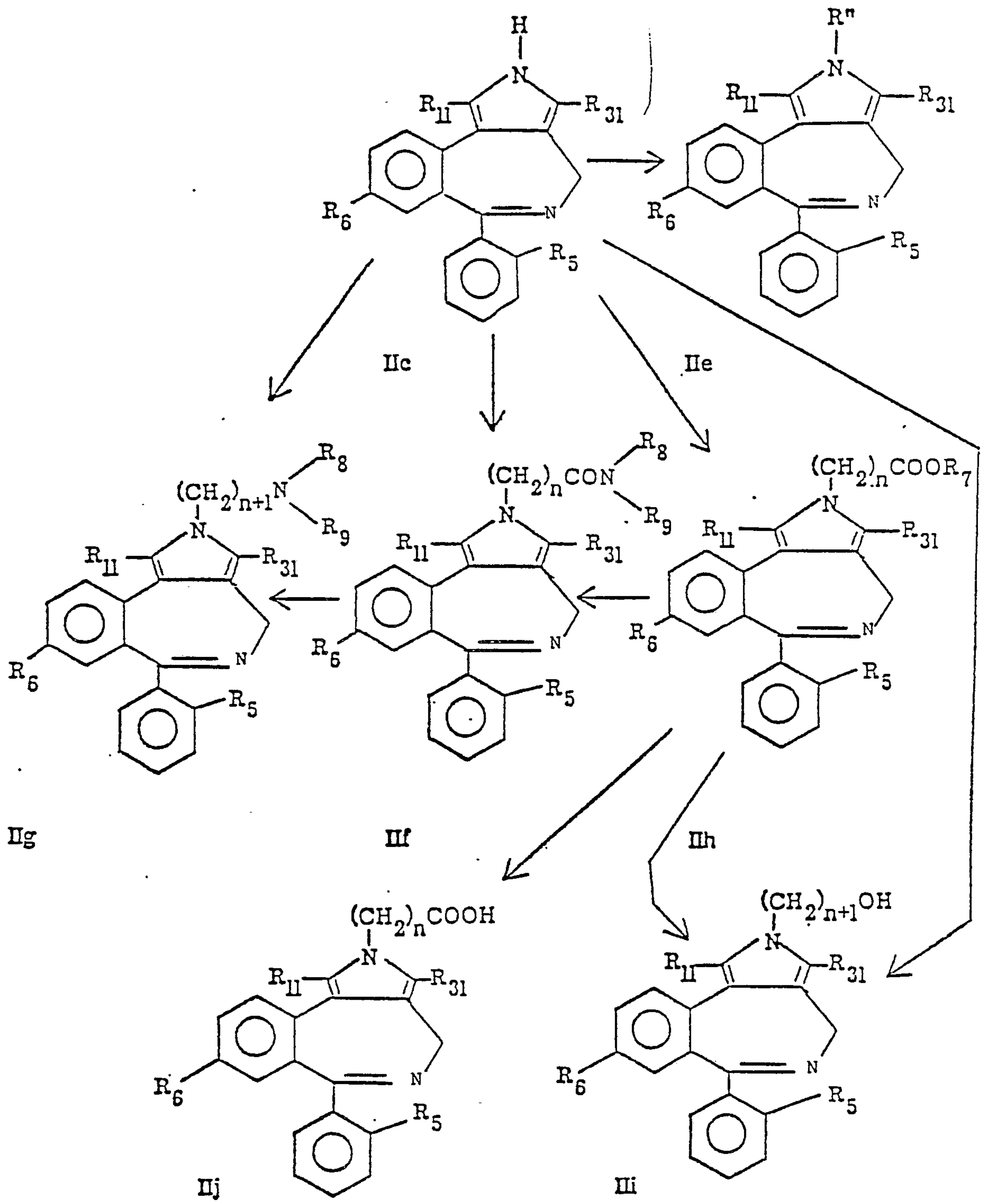

IIc IIe IIg IIf IIh IIj IIi

worin R" ($C_1$-$C_7$)-Alkyl, $R_7$ ($C_1$-$C_7$)-Alkyl, $R_8$ und $R_9$ Wasserstoff oder ($C_1$-$C_7$)-Alkyl und n eine ganze Zahl von 1 bis 6 bedeuten und $R_{11}$, $R_{31}$, $R_5$ und $R_6$ die obige Bedeutung besitzen.

IIc → IIh → IIj

Eine Verbindung der Formel IIc kann in Gegenwart einer Base, wie ein Alkalimetallalkoxid, in einem polaren Lösungsmittel, wie Dimethylsulfoxid oder Dimethylformamid, mit einem Haloester, wie Bromessigsäureäthylester oder 3-Brom-propionsäureäthylester umgesetzt werden. Die Reaktionstemperatur kann in einem Bereich von etwa — 20 °C bis etwa Raumtemperatur liegen, wobei etwa 0 °C bevorzugt ist. Erwünschtenfalls kann das so erhaltene Produkt der Formel IIh in einem wässrigen ätherischen Lösungsmittel, wie Tetrahydrofuran, mit einem Alkalimetall-carbonat oder -hydroxid behandelt werden. Anschliessende Zugabe einer starken Mineralsäure ergibt so die entsprechende Carbonsäure der Formel IIj.

IIh → IIf

Eine Verbindung der Formel IIh kann in einem $C_1$-$C_4$-Alkohol als Lösungsmittel mit Ammoniak oder einem mono- oder di-($C_1$-$C_7$)-Alkylamin und einer katalytischen Menge von dessen Hydrochloridsalz behandelt werden. Die Reaktion erfolgt üblicherweise bei etwa 100 °C unter Verwendung einer Druckapparatur, um die flüchtigen Reaktanten zurückzuhalten.

IIh → IIi

Eine Verbindung der Formel IIh kann in einem ätherischen Lösungsmittel, wie Tetrahydrofuran oder Dioxan, mit einem Metallhydrid, wie Lithiumaluminiumhydrid, umgesetzt werden. Die Reaktionstemperatur kann zwischen etwa — 80 °C und etwa Raumtemperatur liegen, wobei etwa 0 °C bevorzugt ist.

IIc → IIg

Eine Verbindung der Formel IIc kann in Gegenwart einer Base, wie ein Alkalimetallalkoxid, in einem polaren Lösungsmittel, wie Dimethylsulfoxid oder Dimethylformamid, mit einer Verbindung der Formel

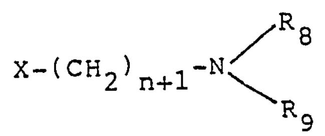

(VI)

worin X ein Halogenid oder Sulfonat bedeutet und $R_8$, $R_9$ und n obige Bedeutung besitzen, umsetzt werden. Die Reaktionstemperatur kann zwischen etwa — 20 °C und etwa Raumtemperatur liegen, wobei etwa Raumtemperatur bevorzugt ist.

IIc → IIe

Eine Verbindung der Formel IIc kann in einem polaren aprotischen Lösungsmittel, wie Dimethylformamid oder Dimethylsulfoxid, mit einer Base, wie ein Alkalimetallalkoxid, z. B. Kalium- oder Natriummethoxid, und anschliessend mit einem Alkylierungsmittel, wie ein ($C_1$-$C_7$)-Alkyl-halogenid oder -sulfonat, umgesetzt werden. Die Reaktionstemperatur kann zwischen etwa 0 °C und etwa Raumtemperatur liegen, wobei etwa 0 °C bevorzugt ist.

IIf → IIg oder IIh → IIi

Eine Verbindung der Formel IIf oder IIh wird in einem ätherischen Lösungsmittel, wie Tetrahydrofuran, mit einem Metallhydrid-Reduktionsmittel, wie Lithiumaluminiumhydrid, umgesetzt. Die Reaktionstemperatur kann zwischen etwa — 20 °C und etwa Raumtemperatur liegen, wobei etwa 0 °C bevorzugt ist.

IIc → IIi

Eine Verbindung der Formel IIc kann in Gegenwart einer Base, wie ein Alkalimetallalkoxid, in Dimethylformamid oder Dimethylsulfoxid mit einer Verbindung der Formel

$$X—(CH_2)_{n+1}OZ \qquad (VII)$$

worin Z eine Hydroxy-Schutzgruppe bedeutet und X und n obige Bedeutung besitzen, umsetzt werden. Geeignete Hydroxy-Schutzgruppen umfassen die Tetrahydropyranyläther-Gruppe. Anschliessende Behandlung mit wässriger Säure ergibt das gewünschte Endprodukt.

IIc → IIf

Eine Verbindung der Formel IIc kann in Gegenwart einer Base, wie ein Alkalimetallalkoxid, in Dimethylformamid oder Dimethylsulfoxid mit einer Verbindung der Formel

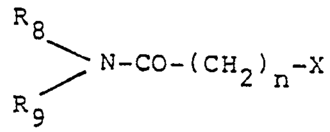 (VIII)

worin X, $R_8$, $R_9$ und n obige Bedeutung besitzen, umsetzt werden.

Die erfindungsgemässen Verbindungen können als Pharmazeutika verwendet werden und besitzen sedative und anxiolytische Eigenschaften. Diese Verbindungen können in Form herkömmlicher pharmazeutischer Präparate verwendet werden ; beispielsweise können die erwähnten Verbindungen mit gebräuchlichen organischen oder anorganischen, inerten, für parenterale oder enterale Verabreichung geeigneten pharmazeutischen Trägern, wie z. B. Wasser, Gelatine, Lactose, Stärke, Magnesiumstearat, Talk, pflanzliche Oele, Gummi arabicum, Polyäthylenglykole, Vaseline® oder dergleichen vermischt werden. Sie können in herkömmlichen pharmazeutischen Formen verabreicht werden, beispielsweise in festen Formen, z. B. Tabletten, Dragées, Kapseln, Suppositorien oder dergleichen, oder in flüssigen Formen, z. B. Lösungen, Suspensionen oder Emulsionen. Darüberhinaus können die erfindungsgemässe Verbindungen enthaltenden pharmazeutischen Präparate herkömmlichen pharmazeutischen Massnahmen, wie Sterilisation, unterworfen werden, und sie können herkömmliche pharmazeutische Hilfsstoffe, wie Konservierungsmittel, Stabilisierungsmittel, Netzmittel, Emulgiermittel, Salze zur Angleichung des osmotischen Drucks oder Puffer, enthalten. Die Präparate können auch andere therapeutisch aktive Substanzen enthalten.

Eine geeignete pharmazeutische Dosierungseinheit kann von etwa 0,1 bis etwa 500 mg der erfindungsgemässen Verbindungen enthalten, wobei ein Dosierungsbereich von etwa 0,1 mg bis etwa 100 mg für orale Verabreichung und ein Dosierungsbereich von etwa 0,1 mg bis etwa 50 mg für parenterale Verabreichung bevorzugt ist. In jedem Einzelfall soll jedoch die spezifische Dosierung nach den individuellen Bedürfnissen und dem fachlichen Urteil der Person, welche die erwähnten Verbindungen verabreicht oder deren Verabreichung überwacht, festgesetzt werden. Es ist zu beachten, dass die obigen Dosierungsangaben lediglich als Beispiele aufzufassen sind und den Umfang und die Anwendbarkeit der vorliegenden Erfindung in keiner Weise einschränken sollen.

Der in dieser Beschreibung verwendete Ausdruck « Dosierungseinheit » bezieht sich auf einzelne pharmazeutische Einheiten, die sich als Einzeldosen für Säuger eignen und von denen jede eine zur Erzielung des therapeutischen Effekts vorbestimmte Menge an Wirkstoff zusammen mit dem benötigten pharmazeutischen Verdünnungsmittel, Träger oder Vehikel enthält.

Die folgenden, unter Verwendung wohlbekannter pharmakologischer Versuche ermittelten Daten zeigen die pharmakologischen Eigenschaften der erfindungsgemässen Verbindungen.

$^3$H-Diazepam-Bindungs-Versuch

Diesen Test verwendet man beim « Screening » auf anxiolytische Eigenschaften.

Nach der von Mohler und Okada (Life Sciences 20, 2 101, 1977) beschriebenen Methode werden Kortex-Fragmente von Rattengehirnen hergestellt und der Bindungs-Versuch durchgeführt, wobei aber anstatt Krebs-Puffer Tris-Puffer verwendet wird. Die Testsubstanzen werden dem Versuch in drei Proben unterworfen. Die Radioaktivität wird mittels Flüssigkeits-Szintillationszählung gemessen. Die Resultate werden als $IC_{50}$ (nM) ausgedrückt, d. h. Angabe der für eine 50 %ige Hemmung der Bindung benötigten Konzentration.

| Aktivität von Vergleichssubstanzen | $IC_{50}$ (nM) |
|---|---|
| Diazepam | 5,0 |
| Flunitrazepam | 1,8 |
| Flurazepam | 15,6 |

| Mit erfindungsgemässen Verbindungen erhaltene Resultate : | $IC_{50}$ (nM) |
|---|---|
| 8-Chlor-6-(2-chlorphenyl)-1,4-dihydro-2-methylpyrrolo[3,4-d][2]benzazepin-3(2H)-on [$LD_{50}$-800 mg/kg (po) Maus] | 0,006 |
| 8-Chlor-6-(2-chlorphenyl)-1,4-dihydro-1-methylpyrrolo[3,4-d][2]benzazepin-3(2H)-on [$LD_{50}$-grösser als 1 000 mg/kg (po) Maus] | 1,0 |
| 8-Chlor-6-(2-chlorphenyl)-1,4-dihydropyrrolo-[3,4-d][2]benzazepin-3(2H)-on, 0,25 molares Hydrat, mikronisiert [$LD_{50}$-grösser als 1 000 mg/kg (po) Maus] | 0,003 |
| 8-Chlor-6-(2-chlorphenyl)-1,4-dihydropyrrolo-[3,4-d][2]benzazepin-3(2H)-on [$LD_{50}$-300 mg/kg (po) Maus] | 0,002 |

Intravenöser Antimetrazol-Test

Dieser Test zeigt antikonvulsive Eigenschaften auf, und man ist der Auffassung, dass er Voraussagen über anxiolytische Aktivitäten erlaubt.

Für diesen Versuch verwendet man männliche, 45-54 Tage alte Mäuse, welche schon während 1 Woche am Ort gehalten wurden und für etwa 24 Stunden kein Futter erhielten. Die Testsubstanzen werden den Tieren in 5 % Akazienöl dispergiert oral verabreicht, wobei pro Dosis drei Tiere verwendet werden. Eine Stunde später wird auf intravenösem Wege Metrazol in einer Dosis von 70 mg/kg verabreicht (konsulsive Dosis 100 mg/kg), und die Tiere werden während 30 Sekunden auf Schutz vor Konvulsionen beobachtet. Die Anzahl der vor Konvulsionen geschützten Tiere wird ermittelt. Die Dosis, bei welcher 50 % der Tiere vor konvulsiven Anfällen geschützt sind, wird als $ED_{50}$ ausgedrückt. Die ungefähre $ED_{50}$ wird nach der Methode von Miller und Tainter (Proc. Soc. Exp. Biol. Med. 57 :261, 1944) berechnet.

| Aktivitäten von Vergleichssubstanzen : | $ED_{50}$ mg/kg po |
|---|---|
| Chlordiazepoxid | 3,9 |
| Diazepam | 1,0 |
| Natrium-Phenobarbital | 19 |

| Mit erfindungsgemässen Verbindungen erhaltene Resultate : | $ED_{50}$ mg/kg po |
|---|---|
| 8-Chlor-6-(2-chlorphenyl)-1,4-dihydro-2-methylpyrrolo[3,4-d][2]benzazepin-3(2H)-on | 0,08 |
| 8-Chlor-6-(2-chlorphenyl)-1,4-dihydro-1-methylpyrrolo[3,4-d][2]benzazepin-3(2H)-on | 0,08 |
| 8-Chlor-6-(2-chlorphenyl)-1,4-dihydropyrrolo-[3,4-d][2]benzazepin-3(2H)-on, 0,25 molares Hydrat, mikronisiert | 0,07 |
| 8-Chlor-6-(2-chlorphenyl)-1,4-dihydropyrrolo-[3,4-d][2]benzazepin-3(2H)-on | 0,03 |

Die folgenden Beispiele sollen die Erfindung illustrieren, ohne sie zu beschränken.

## Beispiel 1

a) Zu einer Lösung von 5,0 g Kupfersulfat in 3 l konzentrierter Ammoniumhydroxidlösung werden in 300 g (4,6 Mol) aktivierter Zinkstaub und 100 g (0,42 Mol) 2-Benzoyl-4-chlorbenzoesäure gegeben. Die Mischung wird während 3 Tagen am Rückfluss gekocht, wobei das Volumen durch Zugabe von konzentrierter Ammoniumhydroxidlösung beibehalten wird. Die Mischung wird abgekühlt, und das überschüssige Zink wird durch Filtration entfernt. Das Filtrat wird durch Zugabe von konzentrieter Salzsäure bis zu einem pH von 3 angesäuert. Der entstehende Niederschlag wird durch Filtration gesammelt und bis zur Gewichtskonstanz getrocknet. Man erhält 2-Benzyl-4-chlorbenzoesäure in Form eines weissen Festkörpers vom Schmelzpunkt 142-144 °C.

b) Zu einer auf 0 °C gekühlten Lösung von 28,4 g (0,75 Mol) Lithiumaluminiumhydrid in 800 ml Aether werden tropfenweise 85,1 g (0,345 mMol) 2-Benzyl-4-chlorbenzoesäure in 250 ml Aether gegeben. Man lässt die Mischung sich auf Raumtemperatur erwärmen, rührt während 2 Stunden und zerstört das überschüssige Lithiumaluminiumhydrid durch Zugabe von 28,5 ml Wasser, 28,5 ml 10 %iger wässriger Natriumhydroxidlösung und 85,5 ml Wasser. Der Niederschlag wird durch Filtration entfernt, und das Filtrat wird über Natriumsulfat getrocknet. Man entfernt den Aether unter vermindertem Druck und erhält 2-Benzyl-4-chlorbenzylalkohol als farbloses Oel, welches beim Stehenlassen kristallisiert ; Schmelzpunkt 46,5-49 °C.

c) Zu einer Suspension von 238 g (1,1 Mol) Pyridiniumchlorchromat und 300 ml Methylenchlorid gibt man 79,3 g (0,34 Mol) 2-Benzyl-4-chlorbenzylalkohol. Die Mischung wird bei Raumtemperatur während 2 Stunden gerührt. Die Chromsalze werden durch Zugabe von 2,4 l einer Mischung von Aether und Petroläther (1 :1) ausgefällt, und der Niederschlag wird durch Filtration durch Celit entfernt. Das Lösungsmittel wird unter vermindertem Druck entfernt, und man erhält 2-Benzyl-4-chlorbenzaldehyd als gelbes Oel, welches ohne Reinigung weiterverarbeitet wird.

d) Zu einer Suspension von 10,5 g (0,437 Mol) von Mineralöl-freiem Natriumhydrid in 1,2 l Tetrahydrofuran werden tropfenweise 58,4 g (0,328 Mol) Diäthylcyanomethylphosphonat gegeben. Nach beendeter Wasserstoffaufnahme (ca. 60 Minuten) werden tropfenweise 69,4 g (0,3 Mol) 2-Benzyl-4-chlorbenzaldehyd in 75 ml Tetrahydrofuran zugegeben. Die Mischung wird über Nacht bei Raumtemperatur gerührt. Die Tetrahydrofuranlösung wird dekantiert und bei Raumtemperatur eingeengt. Der Rückstand wird zwischen 2 l Wasser und 1,5 l Aether verteilt. Die Aetherlösung wird abgetrennt, mit Wasser gewaschen und über Natriumsulfat getrocknet. Der Aether wird unter vermindertem Druck entfernt, und man erhält 3-[2-Benzyl-4-chlorphenyl]-2-propennitril in Form eines gelben Oels, welches ohne Reinigung weiterverarbeitet wird.

e) Eine Mischung von 28,8 g (0,14 Mol) 3-[2-Benzyl-4-chlorphenyl]-2-propennitril, 50 g (0,5 Mol) Chromtrioxid, 100 ml Methylenchlorid und 300 ml Essigsäure wird über Nacht bei Raumtemperatur gerührt. Das überschüssige Chromtrioxid wird durch langsame Zugabe von 30 ml Aethanol zerstört. Die

Mischung wird mit 800 ml Wasser verdünnt und mit 500 ml Aether extrahiert. Die Aetherlösung wird mit Wasser, gesättigtem wässrigem Natriumbicarbonat und gesättigtem wässrigem Natriumchlorid gewaschen, über wasserfreiem Natriumsulfat getrocknet und unter vermindertem Druck eingedampft. Man erhält 3-(2-Benzoyl-4-chlorphenyl)-2-propennitril in Form eines gelbes Oels.

Eine Probe des Produkts wird durch präparative Chromatographie (Kieselgel, 2 mm ; 1 : 1-Mischung von Methylenchlorid und Pentan) gereinigt, und man erhält einen weissen Festkörper vom Schmelzpunkt 87-89 °C.

3-(2-Benzoyl-4-chlorphenyl)-2-propennitril kann auch wie folgt hergestellt werden :

f) Eine Lösung von 92,7 g (0,4 Mol) 2-Amino-5-chlorbenzophenon in 250 ml Acetonitril wird zu einer Mischung von 70 g (0,52 Mol) Kupferchlorid, 65 g (0,63 Mol) t-Butylnitrit, 500 ml Acrylnitril und 500 ml Acetonitril gegeben. Nach beendeter Zugabe wird während 2 Stunden bei Raumtemperatur weitergerührt. Die Mischung wird mit 80 ml 6N Salzsäure und 1 500 ml Wasser verdünnt, worauf man mit Aether extrahiert und über wasserfreiem Natriumsulfat trocknet. Die Aetherlösung wird unter vermindertem Druck konzentriert, und man erhält ein braunes Oel, welches das Endprodukt, α,4-Dichlor-2-(benzoyl)benzolpropannitril, und 2,5-Dichlorbenzophenon enthält. Zerreiben des Oels mit einer Mischung von Aether und Petroläther liefert das Endprodukt als gelbbraunen Festkörper. Umkristallisation einer kleinen Probe des Endprodukts aus einer Mischung von Aether und Petroläther ergibt gelbliche Nadeln vom Schmelzpunkt 69-71 °C.

g) Eine Mischung von 50,9 g (0,168 Mol) α,4-Dichlor-2-(benzoyl)benzolpropannitril, 17 g (0,14 Mol) Kaliumcarbonat, 50,9 g (0,5 Mol) Kaliumbicarbonat und 510 ml Dimethylsulfoxid wird bei Raumtemperatur während 48 Stunden gerührt. Die Mischung wird mit 1,5 l Wasser verdünnt, und der entstandene Niederschlag wird mittels Filtration gesammelt. Umkristallisation aus einer Mischung von Methylenchlorid und Aether gibt 3-(2-Benzoyl-4-chlorphenyl)-2-propennitril als weissliche Prismen vom Schmelzpunkt 89-91 °C.

h) Eine Mischung von 10,7 g (40 mMol) 3-(2-Benzoyl-4-chlorphenyl)-2-propennitril, 5,3 g (38 mMol) Tosymethylisocyanid, 75 ml Dimethylsulfoxid und 150 ml Aether werden tropfenweise zu einer Suspension von 3,7 g (77 mMol) 50 %igem Natriumhydrid in Mineralöl und 170 ml Aether gegeben. Nach beendeter Zugabe wird während 2 Stunden weitergerührt. Die Mischung wird mit Wasser verdünnt, und die Aetherschicht wird abgetrennt. Die wässrige Lösung wird mit Aether extrahiert. Die vereinigten Aetherextrakte werden mit Wasser gewaschen, über wasserfreiem Natriumsulfat getrocknet und unter vermindertem Druck konzentriert, wobei ein dunkelgrünes Oel anfällt. Reinigung durch Säulenchromatographie (800 g Kieselgel ; Elutionsmittel 5 % Aether in Methylenchlorid) ergibt 4-[2-Benzoyl-4-chlorphenyl]-1H-pyrrol-3-carbonitril als weissliche Prismen, Schmelzpunkt 175-177 °C.

i) Eine Mischung von 4,0 g (13 mMol) 4-[2-Benzoyl-4-chlorphenyl]-1H-pyrrol-3-carbonitril, 4 g Raney-Nickel und 300 ml Essigsäure werden in einer Parr-Apparatur während 4 Stunden hydriert. Der Raney-Nickel wird durch Filtration entfernt, und das Filtrat wird mit 400 ml Eiswasser verdünnt. Die Essigsäure wird mit Natriumbicarbonat neutralisiert, und die erhaltene Lösung wird mit Methylenchlorid extrahiert. Die Methylenchlorid-Lösung wird mit Wasser gewaschen und über Natriumsulfat getrocknet. Einengen der Methylenchloridlösung gibt einen gelben Festkörper. Umkristallisation aus Methylenchlorid/Aether liefert 8-Chlor-6-phenyl-2H,4H-pyrrolo[3,4-d][2]benzazepin als weissen Festkörper vom Schmelzpunkt 203-206 °C.

j) In einer Portion werden 4,0 g (13,6 mMol) 8-Chlor-6-phenyl-2H,4H-pyrrolo[3,4-d][2]benzazepin zu einer Mischung von 3,4 g (15,7 mMol) m-Chlorperbenzoesäure in 100 ml einer 2 %igen Lösung von konzentrierter Schwefelsäure in Essigsäure gegeben, und die erhaltene Mischung wird während 1 Stunde gerührt. Die überschüssige Persäure wird durch Zugabe von gesättigter wässriger Natriumbisulfitlösung zerstört, worauf die Mischung unter vermindertem Druck eingeengt wird. Man verteilt den Rückstand zwischen Methylenchlorid und Wasser und neutralisiert mit konzentrierter Ammoniumhydroxidlösung. Die Methylenchloridlösung wird mit Kochsalzlösung gewaschen, über wasserfreiem Natriumsulfat getrocknet und unter vermindertem Druck eingeengt, wobei ein dunkler Rückstand verbleibt. Reinigung des Rückstandes durch Säulenchromatographie (Kieselgel, 100 g ; Eluierungsmittel 5 % Methanol in Methylenchlorid) liefert, nach Umkristallisation aus Essigester, 8-Chlor-1,4-dihydro-6-phenylpyrrolo[3,4-d][2]benzazepin-3(2H)-on als fahlgelbe Prismen vom Schmelzpunkt 205-208 °C.

Dünnschichtchromatographie der Kristallisationsfiltrate weist auf die Anwesenheit des isomeren 8-Chlor-3,4-dihydro-6-phenylpyrrolo[3,4-d][2]benzazepin-1(2H)-ons hin.

Beispiel 2

a) Die Herstellung von α,4-Dichlor-2-(2-fluorbenzoyl)-benzolpropannitril erfolgt in derselben Weise wie die in Beispiel 1f) beschriebene Herstellung von α,4-Dichlor-2-(benzoyl)benzolpropannitril und liefert fahlgelbe Prismen vom Schmelzpunkt 94-95 °C.

b) Die Herstellung von 3-[2-(2-Fluorbenzoyl)-4-chlorphenyl]-2-propennitril erfolgt in derselben Weise wie die in Beispiel 1 g) beschriebene Herstellung von 3-(2-Benzoyl-4-chlorphenyl)-2-propennitril und ergibt weissliche Prismen vom Schmelzpunkt 137-139 °C.

3-[2-(2-Fluorbenzoyl)-4-chlorphenyl]-2-propennitril kann auch wie folgt hergestellt werden :

c) Eine Lösung von 5,0 g (14 mMol) 5-Chlor-2'-fluor-2-jodbenzophenon, 2 ml (14,3 mMol) Triäthylamin, 2 ml (30 mMol) Acrylnitril und 35 mg (1,5 mMol) Palladiumacetat wird unter einer Argonatmosphäre während 16 Stunden am Rückfluss gekocht. Die Mischung wird mit 100 ml 1N Salzsäure verdünnt, worauf der entstehende Niederschlag durch Filtration gesammelt wird. Der Niederschlag wird mit Aether gewaschen und luftgetrocknet ; man erhält 3-[2-(2-Fluor-benzoyl)-4-chlorphenyl]-2-propennitril als weisslichen Festkörper vom Schmelzpunkt 130-133 °C.

d) Die Herstellung von 4-[2-(2-Fluorbenzoyl)-4-chlorphenyl]-1H-pyrrol-3-carbonitril erfolgt in der gleichen Weise wie die in Beispiel 1h) beschriebene Herstellung von 4-[2-Benzoyl-4-chlorphenyl]-1H-pyrrol-3-carbonitril und ergibt weissliche Prismen vom Schmelzpunkt 177-179 °C.

e) Eine Mischung von 3,0 g (9 mMol) 4-[2-(2-Fluorbenzoyl)-4-chlorphenyl]-1H-pyrrol-3-carbonitril, etwa 3 g Raney-Nickel und 150 ml Eisessig wird in einer Parr-Apparatur während 6 Stunden bei 50 psi hydriert. Man entfernt den Raney-Nickel durch Filtration und die Essigsäure unter vermindertem Druck, wobei ein gelbes Oel zurückbleibt. Man giesst dieses gelbe Oel auf Eis, stellt mit Ammoniumhydroxidlösung basisch und extrahiert mit Methylenchlorid. Die Methylenchloridlösung wird über wasserfreiem Natriumsulfat getrocknet und unter vermindertem Druck eingeengt, und man erhält bräunliche Kristalle. Umkristallisation aus einer Mischung von Aether und Methylenchlorid ergibt 8-Chlor-6-(2-fluorphenyl)-2H,4H-pyrrolo[3,4-d][2]benzazepin als crèmefarbige Prismen vom Schmelzpunkt 197-199 °C.

f) In einer Portion werden 5,0 ml (42,1 mMol) einer 30 %-igen Wasserstoffperoxidlösung zu 100 ml einer 1 %igen Lösung von konzentrierter Schwefelsäure in Essigsäure gegeben. Nach 1-stündigem Rühren werden 5,0 g (16,0 mMol) 8-Chlor-6-(2-fluorphenyl)-2H,4H-pyrrolo[3,4-d][2]benzazepin zugegeben, worauf die entstandene Mischung während 4 Stunden gerührt wird. Man zerstört die überschüssige Persäure durch Zugabe von gesättigter wässriger Natriumbisulfitlösung und engt die Mischung unter vermindertem Druck ein. Man verteilt den Rückstand zwischen Methylenchlorid und Wasser und stellt mit konzentrierter Ammoniumhydroxidlösung basisch. Die Methylenchloridlösung wird mit Kochsalzlösung gewaschen, über wasserfreiem Natriumsulfat getrocknet und unter vermindertem Druck eingeengt, wobei 5,0 g eines Rückstands verbleiben. Reinigung durch Hochdruckflüssigkeitschromatographie (Kieselgel, Elutionsmittel 3 % Methanol in Methylenchlorid) gibt als erstes Produkt 8-Chlor-6-(2-fluorphenyl)-3,4-dihydropyrrolo[3,4-d][2]benzazepin-1(2H)-on als fahlgelbe Prismen vom Schmelzpunkt 223-225°C (nach Kristallisation aus Essigester).

Weitere Elution gibt als zweites Produkt 8-Chlor-6-(2-fluorphenyl)-1,4-dihydropyrrolo[3,4-d][2]benzazepin-3(2H)-on als farblose Prismen vom Schmelzpunkt 217-218 °C (nach Kristallisation aus Essigester).

## Beispiel 3

a) Die Herstellung von α,4-Dichlor-2-(2-chlorbenzoyl)-benzolpropannitril erfolgt in derselben Weise wie die in Beispiel 1f) beschriebene Herstellung von α, 4-Dichlor-2-(benzoyl)benzolpropannitril und ergibt weissliche Prismen vom Schmelzpunkt 102-103 °C.

b) Die Herstellung von 3-[2-(2-Chlorbenzoyl)-4-chlorphenyl]-2-propennitril erfolgt in derselben Weise wie die in Beispiel 1g) beschriebene Herstellung von 3-(2-Benzoyl-4-chlorphenyl)-2-propennitril und gibt weissliche Prismen vom Schmelzpunkt 140-141 °C.

c) Die Herstellung von 4-[2-(2-Chlorbenzoyl)-4-chlorphenyl]-1H-pyrrol-3-carbonitril erfolgt in derselben Weise wie die in Beispiel 1h) beschriebene Herstellung von 4-[2-Benzoyl-4-chlorphenyl]-1H-pyrrol-3-carbonitril und gibt weissliche Prismen vom Schmelzpunkt 182-184 °C.

d) Die Herstellung von 8-Chlor-6-(2-chlorphenyl)-2H,4H-pyrrolo[3,4-d][2]benzazepin erfolgt in derselben Weise wie die in Beispiel 2e) beschriebene Herstellung von 8-Chlor-6-(2-fluorphenyl)-2H,4H-pyrrolo[3,4-d][2]benzazepin und ergibt crèmefarbige Prismen vom Schmelzpunkt 204-206 °C.

e) In einer Portion gibt man 10,0 g (30,5 mMol) 8-Chlor-6-(2-chlorphenyl)-2H,4H-pyrrolo[3,4-d][2]benzazepin zu einer Mischung von 7,6 g (35,2 mMol) 80 %iger m-Chlorperbenzoesäure in 225 ml einer 2 %igen Lösung von konzentrierter Schwefelsäure in Essigsäure und rührt während 1 Stunde bei Raumtemperatur. Die überschüssige Persäure wird mit gesättigter wässriger Natriumbisulfitlösung zerstört, worauf die Mischung unter vermindertem Druck eingeengt wird. Man verteilt den Rückstand zwischen Methylenchlorid und Wasser und stellt mit konzentrierter Ammoniumhydroxidlösung basisch. Die Methylenchloridlösung wird mit Kochsalzlösung gewaschen, über wasserfreiem Natriumsulfat getrocknet und unter vermindertem Druck eingedampft, wobei 10 g eines gelben Rückstands verbleiben. Kristallisation aus Methylenchlorid gibt 8-Chlor-6-(2-chlorphenyl)-1,4-dihydropyrrolo[3,4-d][2]-benzazepin-3(2H)-on als fahlgelbe Prismen vom Schmelzpunkt 243-244 °C. Aus der Mutterlauge erhält man durch Kristallisation aus Essigester 8-Chlor-6-(2-chlorphenyl)-3,4-dihydropyrrolo[3,4-d][2]benzazepin-1(2H)-on als fahlgelbe Prismen vom Schmelzpunkt 195-197 °C.

## Beispiel 4

In einer Portion gibt man 9,8 ml 30 %ige Wasserstoffperoxidlösung zu 195 ml einer 1 %igen Lösung von konzentrierter Schwefelsäure in Essigsäure. Die Mischung wird während 1 Stunde bei Raumtemperatur gerührt. Man gibt in einer Portion 9,8 g (30 mMol) 8-Chlor-6-(2-chlorphenyl)-2H,4H-pyrrolo[3,4-d][2]benzazepin zu und rührt die Mischung bei Raumtemperatur während 2,5 Stunden. Die überschüssige Persäure wird durch Zugabe von gesättigter wässriger Natriumbisulfitlösung zerstört, und die Mischung wird unter vermindertem Druck eingeengt. Man verteilt den Rückstand zwischen Methylenchlorid und Wasser und stellt mit konzentrierter Ammoniumhydroxidlösung basisch. Die Methylenchloridlösung wird mit Kochsalzlösung gewaschen, über wasserfreiem Natriumsulfat getrocknet und unter vermindertem Druck eingeengt, wobei 10,0 g eines gelben Rückstands erhalten werden. Reinigung des Rückstands durch Säulenchromatographie (Kieselgel, 200 g ; Eluierungsmittel 5 % Methanol in Methylenchlorid) gibt als erstes Produkt 8-Chlor-6-(2-chlorphenyl)-3,4-dihydropyrrolo[3,4-d][2]benzazepin-1(2H)-on als fahlgelbe Prismen vom Schmelzpunkt 195-197 °C (nach Kristallisation aus Essigester). Weitere Elution gibt als zweites Produkt 8-Chlor-6-(2-chlorphenyl)-1,4-dihydropyrrolo[3,4-d][2]benzazepin-3(2H)-on als farblose Prismen vom Schmelzpunkt 243-244 °C (nach Kristallisation aus Methylenchlorid).

## Beispiel 5

In einer Portion gibt man 15,0 g (69,5 mMol) 80 %ige m-Chlorperbenzoesäure zu einer auf 0 °C gekühlten Lösung von 10,0 g (30,5 mMol) 8-Chlor-6-(2-chlorphenyl)-2H,4H-pyrrolo[3,4-d][2]benzazepin in 350 ml Methylenchlorid. Das Reaktionsgemisch wird während 2,5 Stunden bei 0 °C gerührt und dann mit gesättigter wässriger Natriumbicarbonatlösung neutralisiert. Die Methylenchloridlösung wird mit Wasser gewaschen, über wasserfreiem Natriumsulfat getrocknet und unter vermindertem Druck eingeent, wobei 11,0 g eines gelben Rückstands verbleiben. Reinigung des Rückstands durch Säulenchromatographie (Kieselgel, 150 g ; 5 % Methanol in Methylenchlorid) gibt ein Gemisch von zwei Produkten. Weitere Reinigung des Gemisches durch Chromatographie (Aluminiumoxid, 150 g ; Eluierungsmittel 5 % Methanol in Methylenchlorid) gibt als erstes Produkt 8-Chlor-6-(2-chlorphenyl)-3,4-dihydropyrrolo[3,4-d][2]benzazepin-1(2H)-on-5-oxid als fahlgelbe Prismen vom Schmelzpunkt 243-244 °C (nach Kristallisation aus einer Mischung von Essigester und Methanol).

Weitere Elution gibt als zweites Produkt 8-Chlor-6-(2-chlorphenyl)-1,4-dihydropyrrolo[3,4-d][2]benzazepin-3(2H)-on-5-oxid als fahlgelbe, mit 0,5 Mol Methanol solvatisierte Prismen vom Schmelzpunkt 234-236 °C (nach Kristallisation aus einer Mischung von Essigester und Methanol).

## Beispiel 6

a) Eine Mischung von 33,9 g (0,11 Mol) 3-[2-(2-Chlorbenzoyl)-4-chlorphenyl]-2-propennitril, 20 g (0,96 Mol) 1-Tosyläthylisocyanid in 150 ml Dimethylsulfoxid und 100 ml Aether wird bei Raumtemperatur (Wasserbad) tropfenweise zu einer Suspension von 4,6 g (0,1 Mol) einer 50 %igen Mineralöldispersion von Natriumhydrid in 100 ml Aether gegeben. Man rührt während 2 Stunden bei Raumtemperatur weiter, verdünnt die Mischung mit 1,2 l Wasser und 40 ml 1N Salzsäure und extrahiert mit Methylenchlorid. Die Methylenchloridlösung wird mit Wasser gewaschen, über wasserfreiem Natriumsulfat getrocknet und unter vermindertem Druck eingeengt, wobei ein dunkelgrünes Oel zurückbleibt. Kristallisation aus einer Mischung von Aether und Petroläther gibt 4-[4-Chlor-2-(2-chlorbenzoyl)phenyl]-5-methyl-1H-pyrrol-3-carbonitril als bräunliche Kristalle vom Schmelzpunkt 206-208 °C. Umkristallisation aus Aether gibt das gewünschte Produkt als farblose Kristalle vom Schmelzpunkt 210-211 °C.

Aus Aether erhält man eine zweite Portion von Kristallen, bestehend aus 6-Chlor-8-(2-chlorphenyl)-1,8-dihydro-8-hydroxy-2-methylindeno[2,1-b]pyrrol-3-carbonitril, Schmelzpunkt 221-225 °C. Umkristallisation aus Aether gibt fahlgelbe Prismen vom Schmelzpunkt 232-237 °C.

b) Eine Mischung von 8,5 g (24 mMol) 4-[4-Chlor-2-(2-chlorbenzoyl)phenyl]-5-methyl-1H-pyrrol-3-carbonitril, 1 Löffel Raney-Nickel und 250 ml Eisessig wird über Nacht bei 55 psi in einer Parr-Apparatur hydriert. Man entfernt den Katalysator durch Filtration und die Essigsäure unter vermindertem Druck. Man verdünnt den Rückstand mit Wasser, stellt mit konzentrierter Ammoniumhydroxidlösung basisch und sammelt den entstehenden Niederschlag durch Filtration. Man löst den Niederschlag in Tetrahydrofuran, trocknet über wasserfreiem Natriumsulfat und engt unter vermindertem Druck ein. Durch Kristallisation des Rückstands aus einer Mischung von Aether und Petroläther erhält man weissliche Kristalle vom Schmelzpunkt 219-222 °C. Umkristallisation aus einer Mischung von Aether und Methylenchlorid gibt 8-Chlor-6-(2-chlorphenyl)-1-methyl-2H,4H-pyrrolo[3,4-d][2]benzazepin als farblose Kristalle vom Schmelzpunkt 221-225 °C.

c) In einer Portion gibt man 1 ml 30 %iges Wasserstoffperoxid zu 40 ml einer 1 %igen Lösung von konzentrierter Schwefelsäure in Essigsäure. Nach 1-stündigem Rühren gibt man 2,0 g (5,8 mMol) 8-Chlor-6-(2-chlorphenyl)-1-methyl-2H,4H-pyrrolo[3,4-d][2]benzazepin zu und rührt die entstandene Mischung bei Raumtemperatur während 30 Minuten. Man verdünnt die Mischung mit Methylenchlorid und neutralisiert mit konzentrierter Ammoniumhydroxidlösung. Die Methylenchloridlösung wird über wasserfreiem Natriumsulfat getrocknet und unter vermindertem Druck zu einem dunkelfarbigen Rückstand eingeengt. Kristallisation aus Essigester gibt 8-Chlor-6-(2-chlorphenyl)-1,4-dihydro-1-methyl-pyrrolo[3,4-d][2]benzazepin-3(2H)-on als fahlgelbe Prismen vom Schmelzpunkt 239-241 °C.

## Beispiel 7

a) In einer Portion gibt man 0,7 g (2,2 mMol) 8-Chlor-6-(2-chlorphenyl)-2H,4H-pyrrolo[3,4-d][2]benzazepin zu einer auf 0 °C gekühlten Lösung von 0,3 g (2,6 mMol) Kalium-t-butylat in 30 ml trockenem Dimethylformamid. Nach 15-minütigem Rühren gibt man 1,0 ml (16 mMol) Methyljodid zu und lässt die Mischung sich auf Raumtemperatur erwärmen. Man gibt Wasser zu und extrahiert die Mischung mit Methylenchlorid. Die Methylenchloridlösung wird mit Wasser gewaschen, über wasserfreiem Natriumsulfat getrocknet und unter vermindertem Druck zu einem gelben Oel eingeengt. Reinigung durch Säulenchromatographie (Kieselgel, 20 g ; Eluierungsmittel 5 % Aether in Methylenchlorid) und Umkristallisation aus einer Mischung von Aether und Petroläther gibt 8-Chlor-6-(2-chlorphenyl)-2-methyl-2H,4H-pyrrolo-[3,4-d][2]benzazepin als farblose Prismen vom Schmelzpunkt 167-168 °C.

Das Methansulfonat des 8-Chlor-6-(2-chlorphenyl)-2-methyl-2H,4H-pyrrolo[3,4-d][2]benzazepins wird durch Zugabe äquimolarer Mengen der freien Base und von Methansulfonsäure zu Methanol hergestellt und durch Ausfällung durch Zugabe von Aether isoliert. Umkristallisation aus einer Mischung von Methanol und Aether gibt das Methansulfonat als orangefarbige Prismen vom Schmelzpunkt 200-203 °C.

b) In einer Portion gibt man 1,4 ml 30 %iges Wasserstoffperoxid zu 56 ml einer 1 %igen Lösung von konzentrierter Schwefelsäure in Essigsäure. Nach 1-stündigem Rühren gibt man 2,8 g (8,2 mMol) 8-Chlor-6-(2-chlorphenyl)-2-methyl-2H,4H-pyrrolo[3,4-d][2]benzazepin zu und rührt die entstandene Mischung während 16 Stunden bei Raumtemperatur. Die Mischung wird unter vermindertem Druck eingeengt, und der Rückstand wird zwischen Methylenchlorid und Wasser verteilt. Die Methylenchloridlösung wird mit konzentrierter Ammoniumhydroxidlösung neutralisiert, mit Kochsalzlösung gewaschen, über wasserfreiem Natriumsulfat getrocknet und unter vermindertem Druck zu einem gelben Rückstand eingeengt. Reinigung des Rückstandes durch Säulenchromatographie (Kieselgel, 100 g ; Eluierungsmittel 5 % Methanol in Methylenchlorid) gibt als erstes Produkt 8-Chlor-6-(2-chlorphenyl)-3,4-dihydro-2-methylpyrrolo[3,4-d][2]benzazepin-1(2H)-on als gelbe Prismen vom Schmelzpunkt 177-178 °C (nach Kristallisation aus Aether).

Weitere Elution gibt als zweites Produkt 8-Chlor-6-(2-chlorphenyl)-1,4-dihydro-2-methylpyrrolo[3,4-d][2]-benzazepin-3(2H)-on als fahlgelbe Prismen vom Schmelzpunkt 173-175 °C (nach Kristallisation aus Aether).

## Beispiel 8

a) Eine Lösung von 30,0 g (99,3 mMol) 3-[2-(2-Chlorbenzoyl)-4-chlorphenyl]-2-propennitril und 23,0 g (103 mMol) 1-Tosylpropylisocyanid in einer Mischung von 200 ml Aether und 200 ml Dimethylsulfoxid wird tropfenweise innerhalb von 20 Minuten zu einer eisgekühlten Suspension von 6,7 g (140 mMol) einer 50 %igen Mineralöldispersion von Natriumhydrid in 145 ml Aether gegeben. Nach 1,5-stündigem Rühren tropft man 500 ml Wasser zu und gibt dann 50 ml 1N wässrige Salzsäure zu. Die wässrige Lösung wird mit 3 × 500 ml Aether extrahiert. Die Aetherlösungen werden vereinigt, mit 6 × 800 ml Wasser gewaschen, über wasserfreiem Natriumsulfat getrocknet und unter vermindertem Druck zu 35 g eines dunklen Rückstands eingengt. Reinigung durch Säulen chromatographie (Kieselgel, 500 g ; Eluierungsmittel 50 % Aether in Petroläther) gibt als erstes Produkt 4-[4-Chlor-2-(2-chlorphenyl)phenyl]-5-äthyl-1H-pyrrol-3-carbonitril als weissliche Prismen vom Schmelzpunkt 163-164 °C (nach Kristallisation aus einer Mischung von Aether und Methylenchlorid).

Weitere Elution gibt als zweites Produkt 6-Chlor-8-(2-chlorphenyl)-2-äthyl-1,8-dihydro-8-hydroxyindeno[2,1-b]-pyrrol-3-carbonitril als farblose Prismen vom Schmelzpunkt 172-174 °C (nach Kristallisation aus Aether).

b) Eine Mischung von 3,3 g (8,9 mMol) 4-[2-(2-Chlorbenzoyl)-4-chlorphenyl]-5-äthyl-1H-pyrrol-3-carbonitril, einem halben Teelöffel Raney-Nickel und 100 ml Essigsäure wird bei 50 psi während 16 Stunden in einer Parr-Apparatur hydriert. Man entfernt den Raney-Nickel durch Filtration durch Celit und engt das Filtrat unter vermindertem Druck zu einem dunkelfarbigen Rückstand ein. Man verteilt den Rückstand zwischen Eiswasser und Aether und stellt mit konzentrierter Ammoniumhydroxidlösung basisch. Der Aether wird unter vermindertem Druck entfernt, und der wässrige Rückstand wird während 1 Stunde gerührt. Der entstandene Niederschlag wird durch Filtration gesammelt und in Tetrahydrofuran gelöst. Die Tetrahydrofuranlösung wird über wasserfreiem Natriumsulfat getrocknet und unter vermindertem Druck eingeengt ; der Rückstand kristallisiert aus Aether und ergibt 8-Chlor-6-(2-chlorphenyl)-1-äthyl-2H,4H-pyrrolo[3,4-d][2]benzazepin als gelbe Prismen vom Schmelzpunkt 251-253 °C. Umkristallisation aus einer Mischung von Aether und Methylenchlorid gibt weissliche Prismen vom Schmelzpunkt 254-255 °C.

c) In einer Portion werden 0,9 ml (7,9 mMol) 30 %iges Wasserstoffperoxid zu 40 ml einer 1 %igen Lösung von konzentrierter Schwefelsäure in Essigsäure gegeben. Nach 1-stündigem Rühren gibt man 2,0 g (5,6 mMol) 8-Chlor-6-(2-chlorphenyl)-1-äthyl-2H,4H-pyrrolo[3,4-d][2]benzazepin zu und rührt die entstandene Mischung während 20 Minuten. Die überschüssige Persäure wird durch Zugabe von gesättigter wässriger Natriumbisulfitlösung zerstört, und die Mischung wird unter vermindertem Druck eingeengt. Man verteilt den Rückstand zwischen Methylenchlorid und Wasser und neutralisiert mit konzentrierter Ammoniumhydroxidlösung. Die Methylenchloridlösung wird mit Kochsalzlösung gewaschen, über wasserfreiem Natriumsulfat getrocknet und unter vermindertem Druck eingeengt. Das erhaltene Oel wird aus Aether kristallisiert, und man erhält 8-Chlor-6-(2-chlorphenyl)-1-äthyl-1,4-dihydropyrrolo[3,4-d][2]benzazepin-3(2H)-on als gelbe Prismen vom Schmelzpunkt 233-235 °C. Umkristallisation aus einer Mischung von Essigester und Methanol gibt farblose Prismen vom Schmelzpunkt 236-237 °C.

## Beispiel 9

a) In einer Portion gibt man 2,0 g (5,8 mMol) 8-Chlor-6-(2-chlorphenyl)-1-methyl-2H,4H-pyrrolo[3,4-d][2]benzazepin zu einer eisgekühlten Lösung von 0,8 g (7,1 mMol) Kalium-t-butylat in 20 ml trockenem Dimethylformamid. Nach 10-minütigem Rühren bei 0 °C gibt man 0,8 ml (12,8 mMol) Methyljodid zu und rührt die entstandene Lösung während 20 Minuten. Die Mischung wird mit Wasser verdünnt, und der entstandene Niederschlag wird durch Filtration gesammelt. Man löst den Rückstand in Methylenchlorid, trocknet über wasserfreiem Natriumsulfat und engt unter vermindertem Druck ein, wobei man 8-Chlor-6-(2-chlorphenyl)-1,2-dimethyl-2H,4H-pyrrolo[3,4-d][2]benzazepin als orangefarbige Prismen vom Schmelzpunkt 200-202 °C erhält. Umkristallisation einer Probe aus einer Mischung von Aether und Methylenchlorid gibt fahlgelbe Prismen vom Schmelzpunkt 203-204 °C.

b) In einer Portion gibt man 0,7 ml (5,9 mMol) einer 30 %igen Wasserstoffperoxidlösung zu 33 ml einer 1 %igen Lösung von konzentrierter Schwefelsäure in Essigsäure. Nach 1-stündigem Rühren gibt man 1,8 g (5,0 mMol) 8-Chlor-6-(2-chlorphenyl)-1,2-dimethyl-2H,4H-pyrrolo[3,4-d][2]-benzazepin zu und rührt die entstandene Lösung während 20 Minuten. Man zerstört die überschüssige Persäure durch Zugabe von gesättigter wässriger Natriumbisulfitlösung und engt die Mischung unter vermindertem Druck ein. Man verteilt den Rückstand zwischen Methylenchlorid und Wasser und stellt mit konzentrierter Ammoniumhydroxidlösung basich. Die Methylenchloridlösung wird mit Kochsalzlösung gewaschen, über wasserfreiem Natriumsulfat getrocknet und unter vermindertem Druck zu einem gelben Rückstand eingeengt. Kristallisation aus Essigester gibt 8-Chlor-6-(2-chlorphenyl)-1,4-dihydro-1,2-dimethylpyrrolo[3,4-d][2]-benzazepin-3(2H)-on als fahlgelbe Prismen vom Schmelzpunkt 207-208 °C.

## Beispiel 10

a) In einer Portion gibt man 2,0 g (5,8 mMol) 8-Chlor-6-(2-chlorphenyl)-1-methyl-2H,4H-pyrrolo[3,4-d][2]benzazepin zu einer eisgekühlten Lösung von 0,8 g (7,1 mMol) von Kalium-t-butylat in 30 ml trockenem Dimethylformamid. Nach 15-minütigem Rühren bei 0 °C gibt man 0,65 ml (7,7 mMol) Bromessigsäuremethylester zu und rührt die entstandene Lösung während 5 Minuten. Die Mischung wird mit Wasser verdünnt und mit Aether extrahiert. Die Aetherlösung wird mit Wasser gewaschen, über wasserfreiem Natriumsulfat getrocknet und unter vermindertem Druck zu einem öligen Rückstand eingeengt. Reinigung des Rückstands durch Säulenchromatographie (Kieselgel, 40 g ; Eluierungsmittel 5 % Aether in Methylenchlorid) gibt 8-Chlor-6-(2-chlorphenyl)-1-methyl-2H,4H-pyrrolo[3,4-d][2]benzazepin-2-essigsäuremethylester als weissliche Prismen vom Schmelzpunkt 174-176 °C (nach Kristallisation aus einer Mischung von Aether und Petroläther).

b) In einer Portion gibt man 0,8 ml (7,0 mMol) einer 30 %igen Wasserstoffperoxidlösung zu einer Lösung von 35 ml 1 %iger konzentrierter Schwefelsäure in Essigsäure. Nach 1-stündigem Rühren gibt man 2,0 g (4,8 mMol) 8-Chlor-6-(2-chlorphenyl)-2H,4H-pyrrolo[3,4-d][2]benzazepin-2-essigsäuremethylester zu und rührt die entstandene Lösung während 30 Minuten. Die überschüssige Persäure wird durch Zugabe von gesättigter wässriger Natriumbisulfitlösung zerstört, und die Mischung wird unter vermindertem Druck eingeent. Man verteilt den Rückstand zwischen Methylenchlorid und Wasser und neutralisiert mit konzentrierter Ammoniumhydroxidlösung. Die Methylenchloridlösung wird mit Kochsalzlösung gewaschen, über wasserfreiem Natriumsulfat getrocknet und unter vermindertem Druck eingeengt. Der Rückstand kristallisiert in Essigester und gibt 8-Chlor-6-(2-chlorphenyl)-1,4-dihydro-1-methyl-3-oxo-2H-pyrrolo-[3,4-d][2]benzazepin-2-essigsäuremethylester als fahlgelbe Prismen vom Schmelzpunkt 221-223 °C. Umkristallisation aus Essigester gibt farblose Prismen vom Schmelzpunkt 223-224 °C.

Beispiel 11

a) In einer Portion werden 4,0 g (11,7 mMol) 8-Chlor-6-(2-chlorphenyl)-1-methyl-2H,4H-pyrrolo[3,4-d][2]benzazepin zu einer eisgekühlten Lösung von 1,6 g (14,2 mMol) Kalium-t-butylat in 60 ml trockenem Dimethylformamid gegeben. Nach 15-minütigem Rühren bei 0 °C gibt man 1,4 g (15,0 mMol) 2-Chloracetamid zu und rührt die entstandene Lösung während 5 Minuten. Die Mischung wird mit Wasser verdünnt und mit Methylenchlorid extrahiert. Die Methylenchloridlösung wird mit Wasser gewaschen, über wasserfreiem Natriumsulfat getrocknet und unter vermindertem Druck zu einem roten Rückstand eingeengt. Reinigung des Rückstands durch Säulenchromatographie (Kieselgel, 100 g ; Eluierungsmittel 50 % Aether in Tetrahydrofuran) gibt 8-Chlor-6-(2-chlorphenyl)-1-methyl-2H,4H-pyrrolo[3,4-d][2]benzazepin-2-acetamid als gelben Festkörper. Umkristallisation aus Essigester gibt farblose Nadeln vom Schmelzpunkt 142-145 °C.

b) In einer Portion gibt man 0,7 ml (6,2 mMol) 30 %iges Wasserstoffperoxid zu einer 1 %igen Lösung von konzentrierter Schwefelsäure in Essigsäure. Nach 1-stündigem Rühren gibt man 1,7 g (4,2 mMol) 8-Chlor-6-(2-chlorphenyl)-1-methyl-2H,4H-pyrrolo[3,4-d][2]benzazepin-2-acetamid zu und rührt die entstandene Lösung während 30 Minuten. Die überschüssige Persäure wird durch Zugabe von gesättigter wässriger Natriumbisulfitlösung zerstört, und die Mischung wird unter vermindertem Druck eingeent. Man verteilt den Rückstand zwischen Methylenchlorid und Wasser und neutralisiert mit konzentrierter Ammoniumhydroxidlösung. Die Methylenchloridlösung wird mit Kochsalzlösung gewaschen, über wasserfreiem Natriumsulfat getrocknet und unter vermindertem Druck eingeent. Kristallisation des Rückstands aus Essigester gibt 8-Chlor-6-(2-chlorphenyl)-1,4-dihydro-1-methyl-3-oxo-2H-pyrrolo[3,4-d][2]benzazepin-2-acetamid als farblose Prismen vom Schmelzpunkt 221-222 °C.

Beispiel 12

a) Eine Lösung von 4,0 g (9,6 mMol) 8-Chlor-6-(2-chlorphenyl)-1-methyl-2H,4H-pyrrolo[3,4-d][2]benzazepin-2-essigsäuremethylester in 40 ml Tetrahydrofuran wird tropfenweise zu einer auf — 78 °C gekühlten Lösung von 0,8 g (21,0 mMol) Lithiumaluminiumhydrid in 50 ml Tetrahydrofuran gegeben. Nach beendeter Zugabe lässt man die Reaktionsmischung sich auf 0 °C erwärmen und rührt sie während 30 Minuten. Die Mischung wird mit 1 ml Wasser, 1 ml einer 3N Lösung von wässrigem Natriumhydroxid und 3 ml Wasser behandelt. Der entstehende Niederschlag wird durch Filtration abgetrennt, und das Filtrat wird unter vermindertem Druck eingeent.

Kristallisation des Rückstands aus Aether gibt 8-Chlor-6-(2-chlorphenyl)-1-methyl-2H,4H-pyrrolo[3,4-d][2]benzazepin-2-äthanol als fahlgelbe Prismen vom Schmelzpunkt 149-152 °C. Umkristallisation aus einer Mischung von Aether und Methylenchlorid gibt weisse Prismen vom Schmelzpunkt 151-153 °C.

b) In einer Portion gibt man 0,58 ml (5,1 mMol) 30 %iges Wasserstoffperoxid zu einer 1 %igen Lösung von konzentrierter Schwefelsäure in Essigsäure. Nach 1-stündigem Rühren gibt man 1,3 g (3,3 mMol) 8-Chlor-6-(2-chlorphenyl)-1-methyl-2H,4H-pyrrolo[3,4-d][2]benzazepin-2-äthanol zu und rührt die entstandene Lösung während 30 Minuten. Die überschüssige Persäure wird durch Zugabe von gesättigter wässriger Natriumbisulfitlösung zerstört, und die Mischung wird unter vermindertem Druck eingeengt. Man verteilt den Rückstand zwischen Methylenchlorid und Wasser und neutralisiert mit konzentrierter Ammoniumhydroxidlösung. Die Methylenchloridlösung wird mit Kochsalzlösung gewaschen, über wasserfreiem Natriumsulfat getrocknet und unter vermindertem Druck zu einem gelben Rückstand eingeengt. Reinigung durch Säulenchromatographie (Kieselgel, 30 g ; Eluierungsmittel 3 % Methanol in Methylenchlorid) ergibt als erstes Produkt 2-[2-(Acetoxy)äthyl]-8-chlor-6-(2-chlorphenyl)-1,4-dihydro-1-methylpyrrolo[3,4-d][2]benzazepin-3(2H)-on als farblose Prismen vom Schmelzpunkt 152-154 °C (nach Kristallisation aus einer Mischung von Aether und Methylenchlorid).

Weitere Elution gibt als zweites Produkt 8-Chlor-6-(2-chlorphenyl)-1,4-dihydro-2-(2-hydroxyäthyl)-1-methyl-pyrrolo[3,4-d][2]benzazepin-3(2H)-on als farblose Prismen vom Schmelzpunkt 164-165 °C (nach Kristallisation aus einer Mischung von Essigester und Aether).

Beispiel 13

In einer Portion werden 0,7 g (2 mMol) 8-Chlor-6-(2-chlorphenyl)-3,4-dihydropyrrolo[3,4-d][2]benzazepin-1(2H)-on zu einer eisgekühlten Lösung von 2,6 g (15,8 mMol) Trichloressigsäure und 2,0 g (4,4 mMol) Bleitetraacetat in 30 ml Methylenchlorid zugegeben. Nach 1-stündigem Rühren wird die Mischung mit gesättigter wässriger Natriumbicarbonatlösung neutralisiert. Die Methylenchloridlösung wird mit Wasser gewaschen, über wasserfreiem Natriumsulfat getrocknet und unter vermindertem Druck zu einem gelben Rückstand eingeengt. Reinigung des Rückstands durch Säulenchromatographie

(Kieselgel, 10 g ; Eluierungsmittel 5 % Methanol in Methylenchlorid) und anschliessende Kristallisation aus Aether gibt 8-Chlor-6-(2-chlorphenyl)-3,4-dihydro-3-hydroxypyrrolo[3,4-d][2]benzazepin-1(2H)-on als fahlgelbe Prismen vom Schmelzpunkt 221-222 °C.

Beispiel 14

In einer Portion werden 0,35 g (1 mMol) 8-Chlor-6-(2-chlorphenyl)-1,4-dihydropyrrolo[3,4-d][2]benzazepin-3(2H)-on zu einer eisgekühlten Lösung von 1,3 g (7,9 mMol) Trichloressigsäure und 1,0 g (2,2 mMol) Bleitetraacetat in 15 ml Methylenchlorid gegeben. Nach 4-stündigem Rühren wird die Mischung mit gesättigter wässriger Natriumbicarbonatlösung neutralisiert. Die Methylenchloridlösung wird mit Wasser gewaschen, über wasserfreiem Natriumsulfat getrocknet und unter vermindertem Druck zu einem gelben Rückstand eingeengt. Reinigung des Rückstands durch Säulenchromatographie (Kieselgel, 10 g ; Eluierungsmittel 5 % Methanol in Methylenchlorid) und anschliessende Kristallisation aus Aether gibt 8-Chlor-6-(2-chlorphenyl)-1,4-dihydro-1-hydroxy-pyrrolo[3,4-d][2]benzazepin-3(2H)-on als fahlgelbe Prismen vom Schmelzpunkt 247-249 °C.

Beispiel 15

In einer Portion gibt man 5,0 g (15,2 mMol) 8-Chlor-6-(2-chlorphenyl)-2H,4H-pyrrolo[3,4-d][2]benzazepin zu einer gekühlten Lösung von 20,0 g (120 mMol) Trichloressigsäure und 14,0 g (31,6 mMol) Bleitetraacetat in 50 ml Methylenchlorid. Die Mischung wird während 4 Stunden bei Raumtemperatur gerührt und dann mit Methylenchlorid und Wasser verdünnt. Die Methylenchloridlösung wird mit gesättigter wässriger Natriumbicarbonatlösung gewaschen, über wasserfreiem Natriumsulfat getrocknet und unter vermindertem Druck zu einem grünen Rückstand eingeengt. Reinigung des Rückstands durch Säulenchromatographie (Kieselgel, 100 g ; Eluierungsmittel 5 % Methanol in Methylenchlorid) gibt als erstes Produkt 8-Chlor-6-(2-chlorphenyl)pyrrolo[3,4-d][2]benzazepin-1,3(2H,4H)-dion als gelbe Prismen vom Schmelzpunkt 224-225 °C (nach Kristallisation aus Aether).

Weitere Elution gibt als zweites Produkt 8-Chlor-6-(2-chlorphenyl)-3,4-dihydro-3-hydroxypyrrolo[3,4-d][2]-benzazepin-1(2H)-on als fahlgelbe Prismen vom Schmelzpunkt 222-223 °C (nach Kristallisation aus Aether).

Noch weitere Elution gibt als drittes Produkt 8-Chlor-6-(2-chlorphenyl)-1,4-dihydro-1-hydroxypyrrolo[3,4-d][2]-benzazepin-3(2H)-on als fahlgelbe Prismen vom Schmelzpunkt 247-249 °C (nach Kristallisation aus Aether).

Beispiel 16

12,0 g (27 mMol) Bleitetraacetat werden portionenweise innerhalb von 2 Stunden zu einer Lösung von 6,0 g (12,2 mMol) 8-Chlor-6-(2-chlorphenyl)-2H,4H-pyrrolo[3,4-d]-[2]benzazepin in 120 ml Essigsäure gegeben. Nach Rühren während weiterer 2 Stunden lässt man gasförmigen Schwefelwasserstoff in die Lösung perlen. Der entstehende Niederschlag wird durch Filtration über Celit entfernt. Das Filtrat wird durch Zugabe von gesättigter wässriger Natriumcarbonatlösung neutralisiert und mit Methylenchlorid extrahiert. Die Methylenchloridlösung wird mit Wasser gewaschen, über wasserfreiem Natriumsulfat getrocknet und unter vermindertem Druck zu einem braunen Oel eingeengt. Reinigung durch Säulenchromatographie (Kieselgel, 100 g ; Eluierungsmittel 100 % Methylenchlorid, graduell auf 20 % Aether in Methylenchlorid übergehend) gibt als erstes Produkt einen weisslichen Festkörper. Umkristallisation aus einer Mischung von Methylenchlorid, Aether und Petroläther gibt 8-Chlor-6-(2-chlorphenyl)-2H,4H-pyrrolo[3,4-d][2]benzazepin-1,3-diol-diacetat als farblose Nadeln vom Schmelzpunkt 189-190 °C.

Weitere Elution gibt 1,95 g eines Schaums, welcher aus einer Mischung von Aether und Petroläther kristallisiert ; man erhält 8-Chlor-6-(2-chlorphenyl)-3-acetoxy-3,4-dihydropyrrolo[3,4-d][2]benzazepin-1(2H)-on als farblose Prismen vom Schmelzpunkt 172-174 °C.

Fortgesetzte Elution gibt feine Nadeln. Umkristallisation aus einer Mischung von Aether und Methylenchlorid gibt 8-Chlor-6-(2-chlorphenyl)-1-acetoxy-1,4-dihydropyrrolo[3,4-d]benzazepin-3(2H)-on vom Schmelzpunkt 219-221 °C.

Beispiel 17

Eine Lösung von 0,3 g (0,9 mMol) 8-Chlor-6-(2-chlorphenyl)-3-hydroxy-3,4-dihydropyrrolo[3,4-d][2]benzazepin-1(2H)-on, 0,3 g (1,3 mMol) Benzoesäureanhydrid und 0,3 g (2,5 mMol) Dimethylaminopyridin in einer Mischung von 30 ml Methylenchlorid und 10 ml Tetrahydrofuran wird während 1 Stunde bei 0 °C gerührt. Die Mischung wird mit Wasser verdünnt, nacheinander mit kalter verdünnter Salzsäure, gesättigter wässriger Natriumbicarbonatlösung und Kochsalzlösung gewaschen und über wasserfreiem Natriumsulfat getrocknet. Die Methylenchloridlösung wird unter vermindertem Druck eingeengt. Der Rückstand wird durch Säulenchromatographie (Kieselgel, 10 g ; Eluierungsmittel 5 % Aether in Methylenchlorid) gereinigt, und man erhält 8-Chlor-6-(2-chlorphenyl)-3-(benzoyloxy)-3,4-dihydropyrrolo[3,4-d]-[2]benzazepin-1(2H)-on als farblose Kristalle. Umkristallisation aus Aether gibt farblose Kristalle vom Schmelzpunkt 140-142 °C.

Beispiel 18

Eine Lösung von 0,6 g 8-Chlor-6-(2-chlorphenyl)-3-acetoxy-3,4-dihydropyrrolo[3,4-d][2]benzazepin-1(2H)-on und 3 ml einer 1M methanolischen Lösung von Methansulfonsäure in 10 ml Methanol wird während 30 Minuten bei Raumtemperatur gerührt. Die Lösung wird unter vermindertem Druck auf die Hälfte ihres Volumens eingeengt und mit Aether verdünnt, und der entstehende Niederschlag wird durch Filtration gesammelt, wobei man orangefarbene Kristalle erhält. Umkristallisation aus einer Mischung von Methanol und Aether gibt 8-Chlor-6-(2-chlorphenyl)-3,4-dihydro-3-methoxypyrrolo[3,4-d][2]benzazepin-1(2H)-on-methansulfonat als weissliche Prismen vom Schmelzpunkt 157-160 °C.

Beispiel 19

Eine Lösung von 0,1 g (0,25 mMol) 8-Chlor-6-(2-chlorphenyl)-1-acetoxy-1,4-dihydropyrrolo[3,4-d][2]benzazepin-3(2H)-on und 1 ml einer 1M methanolischen Lösung von Methansulfonsäure wird während 36 Stunden bei Raumtemperatur stehengelassen. Die Mischung wird mit Aether verdünnt. Der entstehende Niederschlag wird durch Filtration gesammelt, und man erhält 8-Chlor-6-(2-chlorphenyl)-1,4-dihydro-1-methoxypyrrolo[3,4-d][2]benzazepin-3(2H)-on-methansulfonat. Umkristallisation aus einer Mischung von Aether und Methanol gibt feine Nadeln vom Schmelzpunkt 185-187 °C.

Beispiel 20

Eine Lösung von 1,5 g (3,4 mMol) 8-Chlor-6-(2-chlorphenyl)-2H,4H-pyrrolo[3,4-d][2]benzazepin-1,3-diol-diacetat in 25 ml einer 1M methanolischen Lösung von Methansulfonsäure wird während 6 Stunden am Rückfluss gekocht. Das Gemisch wird abgekühlt, durch Zugabe von gesättigter wässriger Natriumbicarbonatlösung basisch gestellt und mit Methylenchlorid extrahiert. Die Methylenchloridlösung wird über wasserfreiem Natriumsulfat getrocknet und unter vermindertem Druck eingeengt. Man löst den Rückstand in 4 ml einer 1M methanolischen Lösung von Methansulfonsäure, gibt bis zur Trübung Aether zu und filtriert. Das Filtrat wird mit gesättigtem wässrigem Natriumbicarbonat basisch gestellt. Die Aetherlösung wird über wasserfreiem Natriumsulfat getrocknet und unter vermindertem Druck eingeengt, und man erhält 8-Chlor-6-(2-chlorphenyl)-3,4-dihydro-3-methoxypyrrolo-[3,4-d][2]benzazepin-1(2H)-on als weissliche Kristalle vom Schmelzpunkt 192-194 °C. Die Gegenwart des isomeren 8-Chlor-6(2-chlorphenyl)-1,4-dihydro-1-methoxypyrrolo[3,4-d][2]-benzazepin-3(2H)-on stellt man mittels Dünnschichtchromatographie fest, isoliert es aber nicht.

Das Methansulfonat von 8-Chlor-6-(2-chlorphenyl)-1,4-dihydro-3-methoxypyrrolo[3,4-d][2]benzazepin-1(2H)-on wird durch Lösen der freien Base in einem Ueberschuss methanolischer Methansulfonsäure hergestellt und durch Ausfällung durch Zugabe von Aether isoliert. Umkristallisation aus einer Mischung von Methanol und Aether gibt dieses Salz als farblose Kristalle vom Schmelzpunkt 157-160 °C.

Beispiel 21

In einer Portion gibt man 1,8 ml (20 mMol) Phosphortrichlorid zu einer Lösung von 0,9 g (2,5 mMol) 8-Chlor-6-(2-chlorphenyl)-1,4-dihydropyrrolo[3,4-d][2]benzazepin-3(2H)-on-5-oxid in 180 ml Methylenchlorid. Die Mischung wird während 1 Stunde am Rückfluss gekocht und dann unter vermindertem Druck eingeengt. Man löst den Rückstand in Methylenchlorid und neutralisiert mit gesättigter wässriger Natriumbicarbonatlösung. Die Methylenchloridlösung wird über wasserfreiem Natriumsulfat getrocknet und unter vermindertem Druck eingeengt, und man erhält 8-Chlor-6-(2-chlorphenyl)-1,4-dihydropyrrolo[3,4-d][2]benzazepin-3(2H)-on als orangefarbigen Festkörper. Umkristallisation aus Essigester gibt

fahlgelbe Prismen eines 0,25 molaren Hydrats vom Schmelzpunkt 255-256 °C.

Beispiel 22

In einer Portion gibt man 2 ml (2,3 mMol) Phosphortrichlorid zu einer Lösung von 1,0 g (2,7 mMol) 8-Chlor-6-(2-chlorphenyl)-3,4-dihydropyrrolo[3,4-d][2]benzazepin-1(2H)-on-5-oxid in 200 ml Methylenchlorid und kocht während 1 Stunde am Rückfluss. Man engt die Mischung unter vermindertem Druck ein, löst den Rückstand in Methylenchlorid und neutralisiert mit gesättigter wässriger Natriumbicarbonatlösung. Die Methylenchloridlösung wird über wasserfreiem Natriumsulfat getrocknet und unter vermindertem Druck zu einem dunkelroten Rückstand eingeengt. Reinigung des Rückstands durch Säulenchromatographie (Kieselgel, 20 g ; Eluierungsmittel 5 % Methanol in Methylenchlorid) und anschliessende Kristallisation aus Essigester ergibt 8-Chlor-6-(2-chlorphenyl)-3,4-dihydropyrrolo[3,4-d][2]-benzazepin-1(2H)-on als fahlgeble Prismen, solvatisiert mit 0,5 Mol Essigester, vom Schmelzpunkt 195-197 °C.

Beispiel A

Herstellung von Tabletten (Direkte Verpressung)

| | Bestandteile | mg/Tablette | | | |
|---|---|---|---|---|---|
| 1. | 8-Chlor-6-(2-chlorphenyl)-1,4-dihydropyrrolo[3,4-d]-[2]benzazepin-3(2H)-on oder 8-Chlor-6-(2-chlorphenyl)-1,4-dihydro-1-methylpyrrolo-[3,4-d][2]benzazepin-3(2H)-on | 1 | 5 | 10 | 25 |
| 2. | Lactose | 221 | 217 | 212 | 181 |
| 3. | Avicel | 45 | 45 | 45 | 55 |
| 4. | Stärke für direkte Verpressung | 30 | 30 | 30 | 35 |
| 5. | Magnesiumstearat | 3 | 3 | 3 | 4 |
| | Tablettengewicht | 300 | 300 | 300 | 300 |

Verfahren :

Man vermischt den Wirkstoff gründlich mit der gleichen Menge Lactose, vermischt mit Bestandteilen 3 und 4 und dem Rest der Lactose, gibt dann das Magnesiumstearat zu und mischt während 3 Minuten. Die Masse wird auf einer geeigneten Presse zu Tabletten entsprechender Grösse verpresst.

Beispiel B

Herstellung von Tabletten (Nassgranulierung)

| | Bestandteile | mg/Tablette | | | |
|---|---|---|---|---|---|
| 1. | 8-Chlor-6-(2-chlorphenyl)-1,4-dihydropyrrolo[3,4-d]-[2]benzazepin-3(2H)-on oder 8-Chlor-6-(2-chlorphenyl)-1,4-dihydro-1-methylpyrrolo-[3,4-d][2]benzazepin-3(2H)-on | 1 | 5 | 10 | 25 |
| 2. | Lactose | 202 | 232 | 261 | 280 |

(Fortsetzung)

| | Bestandteile | mg/Tablette | | | |
|---|---|---|---|---|---|
| 3. | Modifizierte Stärke | 25 | 35 | 45 | 55 |
| 4. | Vorgelatinierte Stärke | 20 | 25 | 30 | 35 |
| 5. | Destilliertes Wasser q.s. | - | - | - | - |
| 6. | Magnesiumstearat | 2 | 3 | 4 | 5 |
| | Tablettengewicht | 250 | 300 | 350 | 400 |

Verfahren :

Man vermischt die Bestandteile 1-4 in einem geeigneten Mixer, granuliert mit genügend destilliertem Wasser, um eine geeignete Konsistenz zu erhalten, vermahlt und trocknet in einem geeigneten Ofen. Anschliessend vermahlt man, vermischt während 3 Minuten mit dem Magnesiumstearat und verpresst auf einer geeigneten Presse.

Beispiel C

Herstellung von Kapseln

| | Bestandteile | mg/Kapsel | | | |
|---|---|---|---|---|---|
| 1. | 8-Chlor-6-(2-chlorphenyl)-1,4-dihydropyrrolo[3,4-d]-[2]benzazepin-3(2H)-on oder 8-Chlor-6-(2-chlorphenyl)-1,4-dihydro-1-methylpyrrolo-[3,4-d][2]benzazepin-3(2H)-on | 1 | 5 | 10 | 25 |
| 2. | Lactose | 203 | 193,5 | 328 | 372,5 |
| 3. | Stärke | 30 | 35 | 40 | 30 |
| 4. | Talk | 15 | 15 | 20 | 20 |
| 5. | Aerosol OT | 1 | 1,5 | 2 | 2,5 |
| | Gewicht Kapselinhalt | 250 | 350 | 400 | 450 |

Verfahren :

Man vermischt Bestandteile 1, 2, 3 und 5 in einem geeigneten Mixer, vermahlt, versetzt mit dem Talk und mischt gut. Man füllt auf einer geeigneten Maschine in Kapseln ab.

**Patentansprüche** (für die Vertragsstaaten : BE, CH, DE, FR, GB, IT, LI, LU, NL, SE)

1. Pyrrolo[3,4-d][2]benzazepin-Derivate der allgemeinen Formel

(Siehe Formel Seite 22 f.)

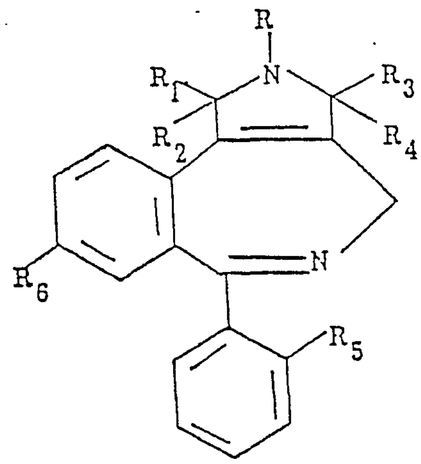

(I)

worin $R_1$ und $R_3$ Wasserstoff, ($C_1$-$C_7$)-Alkyl, Hydroxy, ($C_1$-$C_7$)-Alkoxy oder die Gruppe —OCOR', R' Wasserstoff, ($C_1$-$C_6$)-Alkyl oder Phenyl und $R_2$ und $R_4$ Wasserstoff oder $R_1$ und $R_2$ und/oder $R_3$ und $R_4$ zusammen Oxo-Gruppen bedeuten, mit der Massgabe, dass mindestens eine Oxo-Gruppe vorhanden ist, R Wasserstoff ($C_1$-$C_7$)-Alkyl, Hydroxy-($C_2$-$C_7$)-alkyl, Amino-($C_2$-$C_7$)-alkyl, mono- oder di-($C_1$-$C_7$)-Alkylamino-($C_2$-$C_7$)-alkyl oder die Gruppe -($C_1$-$C_6$)-Alkyl-$COR^{21}$, $R^{21}$ Hydroxy, ($C_1$-$C_7$)-Alkoxy, Amino oder mono- oder di-($C_1$-$C_7$)-Alkylamino, $R_5$ Halogen mit einer 35 nicht übersteigenden Ordnungszahl oder Wasserstoff und $R_6$ Halogen mit einer 35 nicht übersteigenden Ordnungszahl bedeuten, mit der Massgabe, dass wenn $R_1$ oder $R_3$ Hydroxy, ($C_1$-$C_7$)-Alkoxy oder —OCOR' bedeutet, R ($C_1$-$C_7$)-Alkyl oder Wasserstoff bedeutet, die N-Oxide und die pharmazeutisch annehmbaren Salze davon.

2. Verbindungen der in Anspruch 1 definierten allgemeinen Formel I und pharmazeutisch annehmbare Säureadditionssalze davon.

3. Verbindungen gemäss Anspruch 1 oder 2, worin $R_3$ und $R_4$ zusammen eine Oxogruppe bedeuten.

4. Verbindungen gemäss einem der Ansprüche 1-3, worin $R_2$ Wasserstoff bedeutet.

5. Verbindungen gemäss einem der Ansprüche 1-4, worin $R_1$ Wasserstoff oder ($C_1$-$C_7$)-Alkyl bedeutet.

6. Verbindungen gemäss Anspruch 5, worin $R_1$ Wasserstoff oder Methyl bedeutet.

7. Verbindungen gemäss einem der Ansprüche 1-6, worin R Wasserstoff oder ($C_1$-$C_7$)-Alkyl bedeutet.

8. Verbindungen gemäss Anspruch 7, worin R Wasserstoff oder Methyl bedeutet.

9. Verbindungen gemäss einem der Ansprüche 1-8, worin $R_5$ Halogen mit einer 35 nicht übersteigenden Ordnungszahl bedeutet.

10. 8-Chlor-6-(2-chlorphenyl)-1,4-dihydropyrrolo-[3,4-d][2]benzazepin-3(2H)-on.

11. 8-Chlor-6-(2-chlorphenyl)-1,4-dihydro-1-methylpyrrolo[3,4-d][2]benzazepin-3(2H)-on.

12. 8-Chlor-6-(2-chlorphenyl)-1,4-dihydro-2-methylpyrrolo[3,4-d][2]benzazepin-3(2H)-on.

13. 8-Chlor-6-(2-fluorphenyl)-1,4-dihydropyrrolo-[3,4-d][2]benzazepin-3(2H)-on.

14. 8-Chlor-1,4-dihydro-6-phenylpyrrolo[3,4-d][2]benzazepin-3(2H)-on.

15. 8-Chlor-6-(2-chlorphenyl)-1,4-dihydro-1,2-dimethylpyrrolo[3,4-d][2]benzazepin-3(2H)-on.

16. 8-Chlor-6-(2-chlorphenyl)-1,4-dihydro-2-(2-hydroxyäthyl)-1-methylpyrrolo[3,4-d][2]benzazepin-3(2H)-on.

17. 8-Chlor-6-(2-fluorphenyl)-3,4-dihydropyrrolo[3,4-d]-[2]benzazepin-1(2H)-on,
8-Chlor-6-(2-chlorphenyl)-3,4-dihydropyrrolo[3,4-d]-[2]benzazepin-1(2H)-on,
8-Chlor-3,4-dihydro-6-phenylpyrrolo[3,4-d][2]benzazepin-1(2H)-on,
8-Chlor-6-(2-chlorphenyl)-3,4-dihydro-2-methylpyrrolo-[3,4-d][2]benzazepin-1(2H)-on,
8-Chlor-6-(2-chlorphenyl)-1,4-dihydro-1-hydroxypyrrolo[3,4-d][2]benzazepin-3(2H)-on,
8-Chlor-6-(2-chlorphenyl)-3,4-dihydro-3-hydroxypyrrolo[3,4-d][2]benzazepin-1(2H)-on,
8-Chlor-6-(2-chlorphenyl)pyrrolo[3,4-d][2]benzazepin-1,3(2H,4H)-dion,
8-Chlor-6-(2-chlorphenyl)-1,4-dihydropyrrolo[3,4-d]-[2]benzazepin-3(2H)-on-5-oxid,
8-Chlor-6-(2-chlorphenyl)-3,4-dihydropyrrolo[3,4-d]-[2]benzazepin-1(2H)-on-5-oxid,
8-Chlor-6-(2-chlorphenyl)-1-äthyl-1,4-dihydropyrrolo-[3,4-d][2]benzazepin-3(2H)-on,
8-Chlor-6-(2-chlorphenyl)-1,4-dihydro-1-methyl-3-oxo-2H-pyrrolo[3,4-d][2]benzazepin-2-essigsäuremethylester und
8-Chlor-6-(2-chlorphenyl)-1,4-dihydro-1-methyl-3-oxo-2H-pyrrolo[3,4-d][2]benzazepin-2-acetamid.

18. Verbindungen gemäss einem der Ansprüche 1-17 zur Anwendung als therapeutische Wirkstoffe.

19. Verbindungen gemäss einem der Ansprüche 1-17 zur Anwendung als sedative und anxiolytische Wirkstoffe.

20. Verfahren zur Herstellung von Verbindungen gemäss einem der Ansprüche 1-17, dadurch gekennzeichnet, dass man

a) zur Herstellung von Verbindungen der in Anspruch 1 definierten allgemeinen Formel I, worin $R_1$ oder $R_3$ Wasserstoff oder ($C_1$-$C_7$)-Alkyl bedeutet und die übrigen Symbole die in Anspruch 1 angegebene Bedeutung besitzen, und von N-Oxiden davon, eine Verbindung der allgemeinen Formel

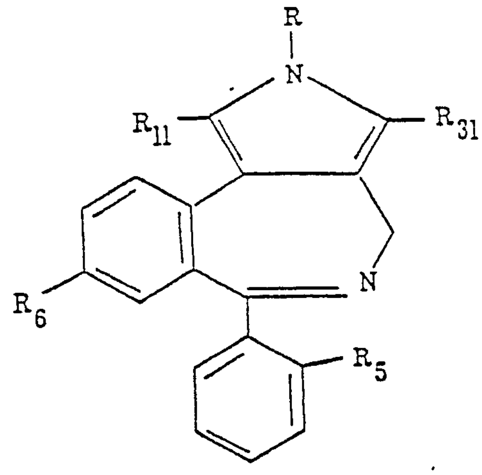

(II)

worin eines von $R_{11}$ und $R_{31}$ Wasserstoff und das andere Wasserstoff oder ($C_1$-$C_7$)-Alkyl bedeutet und R, $R_5$ und $R_6$ die in Anspruch 1 angegebene Bedeutung besitzen, mit einer Persäure oxidiert, oder

b) zur Herstellung von Verbindungen der in Anspruch 1 definierten allgemeinen Formel I, worin $R_1$ oder $R_3$ Hydroxy bedeutet und die übrigen Substituenten die in Anspruch 1 angegebene Bedeutung besitzen oder $R_1$ und $R_2$ und $R_3$ und $R_4$ jeweils zusammen Oxogruppen bedeuten und die übrigen Symbole die in Anspruch 1 angegebene Bedeutung besitzen, eine Verbindung der in Anspruch 1 definierten Formel I, worin $R_1$ oder $R_3$ Wasserstoff bedeutet und die übrigen Substituenten die in Anspruch 1 angegebene Bedeutung besitzen, oder eine Verbindung der obigen Formel II, worin $R_{11}$ und $R_{31}$ Wasserstoff bedeuten, mit einem Blei-(IV)-carboxylat in Gegenwart einer starken Säure oxidiert oder

c) zur Herstellung von Verbindungen der in Anspruch 1 definierten Formel I, worin $R_1$ oder $R_3$ Acetoxy bedeutet und die übrigen Substituenten die in Anspruch 1 angegebene Bedeutung besitzen, eine Verbindung der in Anspruch 1 definierten Formel I, worin $R_1$ oder $R_3$ Wasserstoff bedeutet und die übrigen Substituenten die in Anspruch 1 angegebene Bedeutung besitzen, oder eine Verbindung der obigen Formel II, worin $R_{11}$ und $R_{31}$ Wasserstoff bedeuten, mit Blei-tetraacetat in Essigsäure behandelt oder

d) zur Herstellung von Verbindungen der in Anspruch 1 definierten Formel I, worin $R_1$ oder $R_3$ —OCOR′ bedeutet und die übrigen Substituenten die in Anspruch 1 angegebene Bedeutung besitzen, eine Verbindung der in Anspruch 1 definierten Formel I, worin $R_1$ oder $R_3$ Hydroxy bedeutet und die übrigen Substituenten die in Anspruch 1 angegebene Bedeutung besitzen, in Gegenwart einer Base mit einem Carbonsäureanhydrid behandelt oder

e) zur Herstellung von Verbindungen der in Anspruch 1 definierten Formel I, worin $R_1$ oder $R_3$ ($C_1$-$C_7$)-Alkoxy bedeutet und die übrigen Substituenten die in Anspruch 1 angegebene Bedeutung besitzen, eine Verbindung der in Anspruch 1 definierten Formel I, worin $R_1$ oder $R_3$ -OCOR′ oder Hydroxy bedeutet und die übrigen Substituenten die in Anspruch 1 angegebene Bedeutung besitzen, oder eine Verbindung der Formel

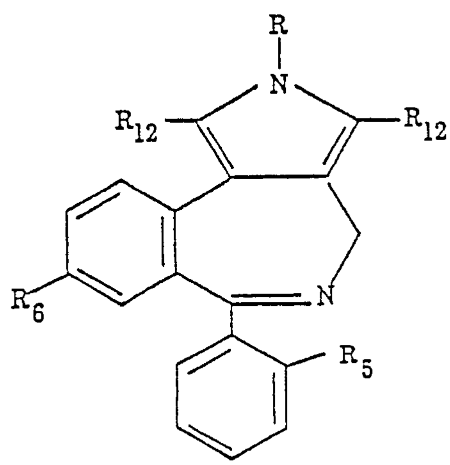

(III)

worin $R_{12}$ Acetoxy bedeutet und R, $R_5$ und $R_6$ die in Anspruch 1 angegebene Bedeutung besitzen, in Gegenwart einer starken Säure mit einem ($C_1$-$C_7$)- Alkanol behandelt oder

f) zur Herstellung von Verbindungen der in Anspruch 1 definiernten Formel I, worin $R_1$ oder $R_3$ Wasserstoff oder ($C_1$-$C_7$)-Alkyl bedeutet und die übrigen Substituenten die in Anspruch 1 angegebene Bedeutung besitzen, ein N-Oxid der Formel

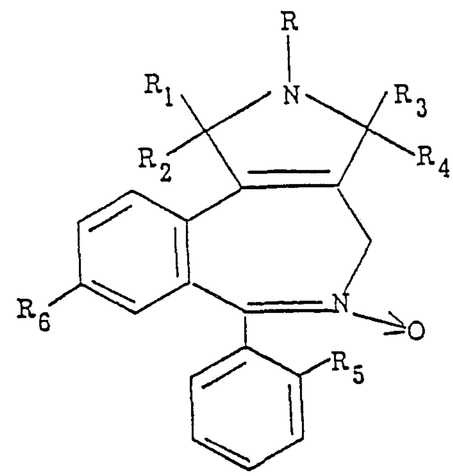

(IA)

worin R, $R_1$, $R_2$, $R_3$, $R_4$, $R_5$ und $R_6$ die in Anspruch 1 angegebene Bedeutung besitzen, desoxidiert und erwünschtenfalls

g) eine Verbindung der Formel I in ein pharmazeutisch annehmbares Salz oder ein pharmazeutisch annehmbares Salz einer Verbindung der Formel I in ein anderes pharmazeutisch annehmbares Salz überführt.

21. Arzneimittel, enthaltend eine Verbindung gemäss einem der Ansprüche 1-17 und ein therapeutisch inertes Excipiens.

22. Sedatives und anxiolytisches Mittel, enthaltend eine Verbindung gemäss einem der Ansprüche 1-17 und ein therapeutisch inertes Excipiens.

**Patentansprüche** (für den Vertragsstaat AT)

1. Verfahren zur Herstellung von Pyrrolo [3,4-d][2]-benzazepin-Derivaten der allgemeinen Formel

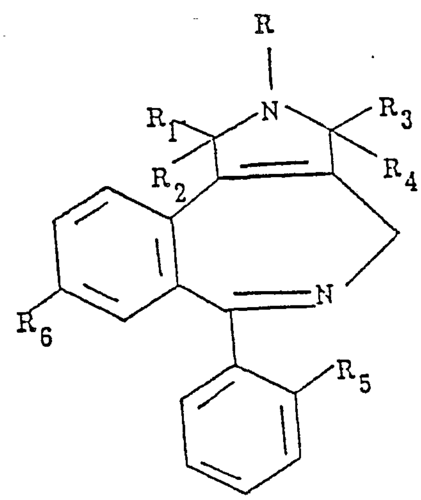

(I)

worin $R_1$ und $R_3$ Wasserstoff, ($C_1$-$C_7$)-Alkyl, Hydroxy, ($C_1$-$C_7$)-Alkoxy oder die Gruppe —OCOR', R' Wasserstoff, ($C_1$-$C_6$)-Alkyl oder Phenyl und $R_2$ und $R_4$ Wasserstoff oder $R_1$ und $R_2$ und/oder $R_3$ und $R_4$ zusammen Oxo-Gruppen bedeuten, mit der Massgabe, dass mindestens eine Oxo-Gruppe vorhanden ist, R Wasserstoff, ($C_1$-$C_7$)-Alkyl, Hydroxy-($C_2$-$C_7$)-alkyl, Amino-($C_2$-$C_7$)-Alkyl oder mono- oder di-($C_1$-$C_7$)-Alkylamino-($C_2$-$C_7$)-alkyl oder die Gruppe -($C_1$-$C_6$)-Alkyl-$COR^{21}$,. $R^{21}$ Hydroxy, ($C_1$-$C_7$)-Alkoxy, Amino oder mono- oder di-($C_1$-$C_7$)-Alkylamino, $R^5$ Halogen mit einer 35 nicht übersteigenden Ordnungszahl oder Wasserstoff und $R_6$ Halogen mit einer 35 nicht übersteigenden Ordnungszahl bedeuten, mit der Massgabe, dass wenn $R_1$ oder $R_3$ Hydroxy, ($C_1$-$C_7$)-Alkoxy oder —OCOR' bedeutet, R ($C_1$-$C_7$)-Alkyl oder Wasserstoff bedeutet, von N-Oxiden und von pharmazeutisch annehmbaren Salzen davon, dadurch gekennzeichnet, dass man

a) zur Herrstellung von Verbindungen der obigen Formel I, worin $R_1$ oder $R_3$ Wasserstoff oder ($C_1$-$C_7$)-Alkyl bedeutet und die übrigen Substituente die obige Bedeutung besitzen, und von N-Oxiden davon, eine Verbindung der allgemeinen Formel

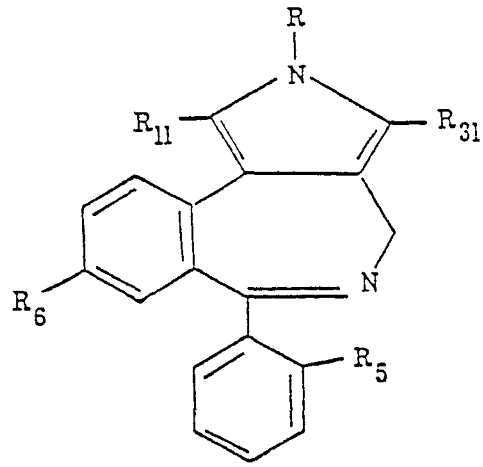

(II)

worin eines von $R_{11}$ und $R_{31}$ Wasserstoff und das andere Wasserstoff oder ($C_1$-$C_7$)-Alkyl bedeutet und R, $R_5$ und $R_6$ obige Bedeutung besitzen, mit einer Persäure oxidiert, oder

b) zur Herstellung von Verbindungen der obigen Formel I, worin $R_1$ oder $R_3$ Hydroxy bedeutet und die übrigen Substituenten die obige Bedeutung besitzen oder worin $R_1$ und $R_2$ und $R_3$ und $R_4$ jeweils zusammen Oxogruppen bedeuten und die übrigen Symbole die obige Bedeutung besitzen, eine Verbindung der Formel I, worin $R_1$ oder $R_3$ Wasserstoff bedeutet und die übrigen Substituenten die obige Bedeutung besitzen, oder eine Verbindung der obigen Formel II, worin $R_{11}$ und $R_{31}$. Wasserstoff bedeuten, mit einem Blei-(IV)-carboxylat in Gegenwart einer starken Säure oxidiert oder

c) zur Herstellung von Verbindungen der obigen Formel I, worin $R_1$ oder $R_3$ Acetoxy bedeutet und die übrigen Substituenten die obige Bedeutung besitzen, eine Verbindung der Formel I, worin $R_1$ oder $R_3$ Wasserstoff bedeutet und die übrigen Substituenten die obige Bedeutung besitzen, oder eine Verbindung der obigen Formel II, worin $R_{11}$ und $R_{31}$ Wasserstoff bedeuten, mit Blei-tetraacetat in Essigsäure behandelt oder

d) zur Herstellung von Verbindungen der obigen Formel I, worin $R_1$ oder $R_3$ —OCOR' bedeutet und die übrigen Substituenten die obige Bedeutung besitzen, eine Verbindung der Formel I, worin $R_1$ oder $R_3$ Hydroxy bedeutet und die übrigen Substituenten die obige Bedeutung besitzen, in Gegenwart einer Base mit einem Carbonsäureanhydrid behandelt oder

e) zur Herstellung von Verbindungen der obigen Formel I, worin $R_1$ oder $R_3$ ($C_1$-$C_7$)-Alkoxy bedeutet und die übrigen Substituenten die obige Bedeutung besitzen, eine Verbindung der Formel I, worin $R_1$ oder $R_3$ —OCOR' oder Hydroxy bedeutet und die übrigen Substituenten die obige Bedeutung besitzen, oder eine Verbindung der Formel

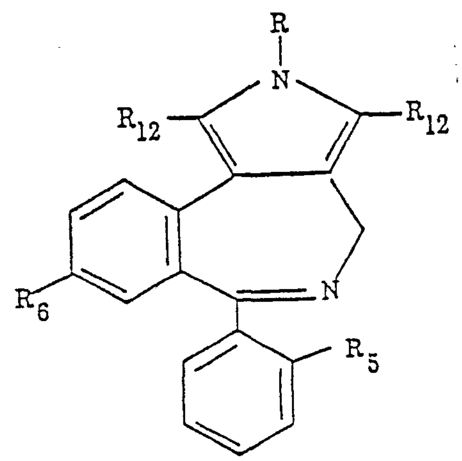

(III)

worin $R_{12}$ Acetoxy bedeutet und R, $R_5$ und $R_6$ die obige Bedeutung besitzen, in Gegenwart einer starken Säure mit einem ($C_1$-$C_7$)-Alkanol behandelt oder

f) zur Herstellung von Verbindungen der obigen Formel I, worin $R_1$ oder $R_3$ Wasserstoff oder ($C_1$-$C_7$)-Alkyl bedeutet und die übrigen Substituenten die obige Bedeutung besitzen, ein N-Oxid der allgemeinen Formel

(Siehe Formel Seite 26 f.)

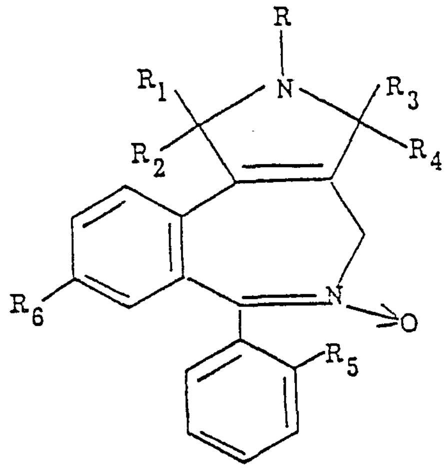

(IA)

worin R, $R_1$, $R_2$, $R_3$, $R_4$, $R_5$ und $R_6$ obige Bedeutung besitzen, desoxidiert und erwünschtenfalls

g) eine Verbindung der Formel I in ein pharmazeutisch annehmbares Salz oder ein pharmazeutisch annehmbares Salz einer Verbindung der Formel I in ein anderes pharmazeutisch annehmbares Salz überführt.

2. Verfahren gemäss Anspruch 1, dadurch gekennzeichnet, dass man eine Verbindung der in Anspruch 1 definierten Formel I oder ein pharmazeutisch annehmbares Säureadditionssalz davon herstellt.

3. Verfahren gemäss Anspruch 1 oder 2, dadurch gekennzeichnet, dass $R_3$ und $R_4$ zusammen eine Oxogruppe bedeuten.

4. Verfahren gemäss einem der Ansprüche 1-3, dadurch gekennzeichnet, dass $R_2$ Wasserstoff bedeutet.

5. Verfahren gemäss einem der Ansprüche 1-4, dadurch gekennzeichnet, dass $R_1$ Wasserstoff oder ($C_1$-$C_7$)-Alkyl bedeutet.

6. Verfahren gemäss Anspruch 5, dadurch gekennzeichnet, dass $R_1$ Wasserstoff oder Methyl bedeutet.

7. Verfahren gemäss einem der Ansprüche 1-6, dadurch gekennzeichnet, dass R Wasserstoff oder ($C_1$-$C_7$)-Alkyl bedeutet.

8. Verfahren gemäss Anspruch 7, dadurch gekennzeichnet, dass R Wasserstoff oder Methyl bedeutet.

9. Verfahren gemäss einem der Ansprüche 1-8, dadurch gekennzeichnet, dass $R_5$ Halogen mit einer 35 nicht übersteigenden Ordnungszahl bedeutet.

10. Verfahren gemäss Anspruch 1, dadurch gekennzeichnet, dass man 8-Chlor-6-(2-chlorphenyl)-1,4-dihydropyrrolo-[3,4-d][2]benzazepin-3(2H)-on herstellt.

11. Verfahren gemäss Anspruch 1, dadurch gekennzeichnet, dass man 8-Chlor-6-(2-chlorphenyl)-1,4-dihydro-1-methylpyrrolo[3,4-d][2]benzazepin-3(2H)-on herstellt.

12. Verfahren gemäss Anspruch 1, dadurch gekennzeichnet, dass man 8-Chlor-6-(2-chlorphenyl)-1,4-dihydro-2-methylpyrrolo[3,4-d][2]benzazepin-3(2H)-on herstellt.

13. Verfahren gemäss Anspruch 1, dadurch gekennzeichnet, dass man 8-Chlor-6-(2-fluorphenyl)-1,4-dihydropyrrolo[3,4-d][2]benzazepin-3(2H)-on herstellt.

14. Verfahren gemäss Anspruch 1, dadurch gekennzeichnet, dass man 8-Chlor-1,4-dihydro-6-phenylpyrrolo-[3,4-d][2]benzazepin-3(2H)-on herstellt.

15. Verfahren gemäss Anspruch 1, dadurch gekennzeichnet, dass man 8-Chlor-6-(2-chlorphenyl)-1,4-dihydro-1,2-dimethylpyrrolo[3,4-d][2]benzazepin-3(2H)-on herstellt.

16. Verfahren gemäss Anspruch 1, dadurch gekennzeichnet, dass man 8-Chlor-6-(2-chlorphenyl)-1,4-dihydro-2-(2-hydroxyäthyl)-1-methylpyrrolo[3,4-d][2]benzazepin-3(2H)-on herstellt.

**Claims** (for the Contracting States : BE, CH, DE, FR, GB, IT, LI, LU, NL, SE)

1. Pyrrolo[3,4-d][2]benzazepine derivatives of the general formula

(See formula page 27)

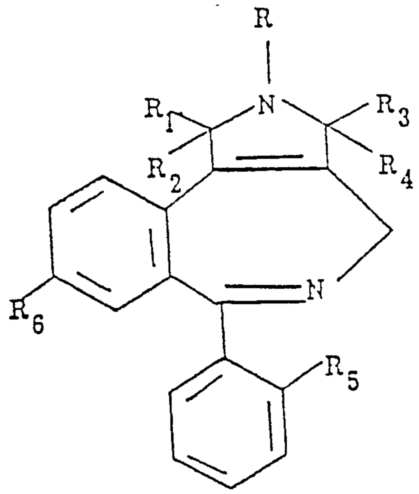

(I)

wherein $R_1$ and $R_3$ signify hydrogen, ($C_1$-$C_7$)-alkyl, hydroxy, ($C_1$-$C_7$)-alkoxy or the group —OCOR', R' signifies hydrogen, ($C_1$-$C_6$)-alkyl or phenyl and $R_2$ and $R_4$ signify hydrogen or $R_1$ and $R_2$ and/or $R_3$ and $R_4$ together signify oxo groups, with the proviso that at least one oxo group is present, R signifies hydrogen, ($C_1$-$C_7$)-alkyl, hydroxy-($C_2$-$C_7$)-alkyl, amino-($C_2$-$C_7$)-alkyl, mono- or di-($C_1$-$C_7$)-alkylamino-($C_2$-$C_7$)-alkyl or the group -($C_1$-$C_6$)-alkyl-$COR^{21}$, $R^{21}$ signifies hydroxy, ($C_1$-$C_7$)-alkoxy, amino or mono- or di-($C_1$-$C_7$)-alkylamino, $R_5$ signifies halogen with an atomic number not exceeding 35 or hydrogen and $R_6$ signifies halogen with an atomic number not exceeding 35, with the proviso that R signifies ($C_1$-$C_7$)-alkyl or hydrogen when $R_1$ or $R_3$ signifies hydroxy, ($C_1$-$C_7$)-alkoxy or —OCOR', the N-oxides and the pharmaceutically acceptable salts thereof.

2. Compounds of general formula I defined in claim 1 and pharmaceutically acceptable acid addition salts thereof.

3. Compounds in accordance with claim 1 or 2, wherein $R_3$ and $R_4$ together signify an oxo group.

4. Compounds in accordance with any one of claims 1-3, wherein $R_2$ signifies hydrogen.

5. Compounds in accordance with any one of claims 1-4, wherein $R_1$ signifies hydrogen or ($C_1$-$C_7$)-alkyl.

6. Compounds in accordance with claim 5, wherein $R_1$ signifies hydrogen or methyl.

7. Compounds in accordance with any one of claims 1-6, wherein R signifies hydrogen or ($C_1$-$C_7$)-alkyl.

8. Compounds in accordance with claim 7, wherein R signifies hydrogen or methyl.

9. Compounds in accordance with any one of claims 1-8, wherein $R_5$ signifies halogen with an atomic number not exceeding 35.

10. 8-Chloro-6-(2-chlorophenyl)-1,4-dihydropyrrolo-[3,4-d][2]benzazepin-3(2H)-one.

11. 8-Chloro-6-(2-chlorophenyl)-1,4-dihydro-1-methylpyrrolo[3,4-d][2]benzazepin-3(2H)-one.

12. 8-Chloro-6-(2-chlorophenyl)-1,4-dihydro-2-methylpyrrolo[3,4-d][2]benzazepin-3(2H)-one.

13. 8-Chloro-6-(2-fluorophenyl)-1,4-dihydropyrrolo-[3,4-d][2]benzazepin-3(2H)-one.

14. 8-Chloro-1,4-dihydro-6-phenylpyrrolo[3,4-d][2]-benzazepin-3(2H)-one.

15. 8-Chloro-6-(2-chlorophenyl)-1,4-dihydro-1,2-dimethylpyrrolo[3,4-d][2]benzazepin-3(2H)-one.

16. 8-Chloro-6-(2-chlorophenyl)-1,4-dihydro-2-(2-hydroxyethyl)-1-methylpyrrolo[3,4-d][2]benzazepin-3(2H)-one.

17. 8-Chloro-6-(2-fluorophenyl)-3,4-dihydropyrrolo-[3,4-d][2]benzazepin-1(2H)-one,
8-chloro-6-(2-chlorophenyl)-3,4-dihydropyrrolo[3,4-d]-[2]benzazepin-1(2H)-one,
8-chloro-3,4-dihydro-6-phenylpyrrolo[3,4-d][2]benzazepin-1(2H)-one,
8-chloro-6-(2-chlorophenyl)-3,4-dihydro-2-methylpyrrolo-[3,4-d][2]benzazepin-1(2H)-one,
8-chloro-6-(2-chlorophenyl)-1,4-dihydro-1-hydroxypyrrolo[3,4-d][2]benzazepin-3(2H)-one,
8-chloro-6-(2-chlorophenyl)-3,4-dihydro-3-hydroxypyrrolo[3,4-d]benzazepin-1(2H)-one,
8-chloro-6-(2-chlorophenyl)pyrrolo[3,4-d][2]benzazepine-1,3-(2H,4H)-dione,
8-chloro-6-(2-chlorophenyl)-1,4-dihydropyrrolo[3,4-d]-[2]benzazepin-3(2H)-one-5-oxide,
8-chloro-6-(2-chlorophenyl)-3,4-dihydropyrrolo[3,4-d]-[2]benzazepin-1(2H)-one-5-oxide,
8-chloro-6-(2-chlorophenyl)-1-ethyl-1,4-dihydropyrrolo-[3,4-d][2]benzazepin-3(2H)-one,
8-chloro-6-(2-chlorophenyl)-1,4-dihydro-1-methyl-3-oxo-2H-pyrrolo[3,4-d][2]benzazepine-2-acetic acid methyl ester and
8-chloro-6-(2-chlorophenyl)-1,4-dihydro-1-methyl-3-oxo-2H-pyrrolo[3,4-d][2]benzazepine-2-acetamide.

18. Compounds in accordance with any one claims 1-17 for use as therapeutically active substances.

19. Compounds in accordance with any one of claims 1-17 for use as sedative and anxiolytic active substances.

20. A process for the manufacture of compounds in accordance with any one of claims 1-17, characterized by

a) for the manufacture of compounds of general formula I defined in claim 1 in which $R_1$ or $R_3$ signifies hydrogen or $(C_1\text{-}C_7)$-alkyl and the remaining symbols have the significance given in claim 1 and of N-oxides thereof, oxidizing a compound of the general formula

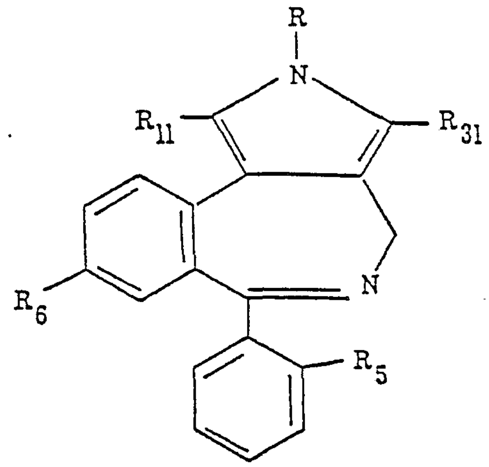

(II)

wherein one of $R_{11}$ and $R_{31}$ signifies hydrogen and the other signifies hydrogen or $(C_1\text{-}C_7)$-alkyl and R, $R_5$ and $R_6$ have the significance given in claim 1, with a peracid, or

b) for the manufacture of compounds of general formula I defined in claim 1 in which $R_1$ or $R_3$ signifies hydroxy and the remaining substituents have the significance given in claim 1 or $R_1$ and $R_2$ and $R_3$ and $R_4$ in each case together signify oxo groups and the remaining symbols have the significance given in claim 1, oxidizing a compound of formula I defined in claim 1 in which $R_1$ or $R_3$ signifies hydrogen and the remaining substituents have the significance given in claim 1 or a compound of formula II above in which $R_{11}$ and $R_{31}$ signify hydrogen with a lead (IV) carboxylate in the presence of a strong acid, or

c) for the manufacture of compounds of formula I defined in claim 1 in which $R_1$ or $R_3$ signifies acetoxy and the remaining substituents have the significance given in claim 1, treating a compound of formula I defined in claim 1 in which $R_1$ or $R_3$ signifies hydrogen and the remaining substituents have the significance given in claim 1 or a compound of formula II above in which $R_{11}$ and $R_{31}$ signify hydrogen with lead tetraacetate in acetic acid, or

d) for the manufacture of compounds of formula I defined in claim 1 in which $R_1$ or $R_3$ signifies —OCOR' and the remaining substituents have the significance given in claim 1, treating a compound of formula I defined in claim 1 in which $R_1$ or $R_3$ signifies hydroxy and the remaining substituents have the significance given in claim 1 with a carboxylic acid anhydride in the presence of a base, or

e) for the manufacture of compounds of formula I defined in claim 1 in which $R_1$ or $R_3$ signifies $(C_1\text{-}C_7)$-alkoxy and the remaining substituents have the significance given in claim 1, treating a compound of formula I defined in claim 1 in which $R_1$ or $R_3$ signifies —OCOR' or hydroxy and the remaining substituents have the significance given in claim 1 or a compound of the formula

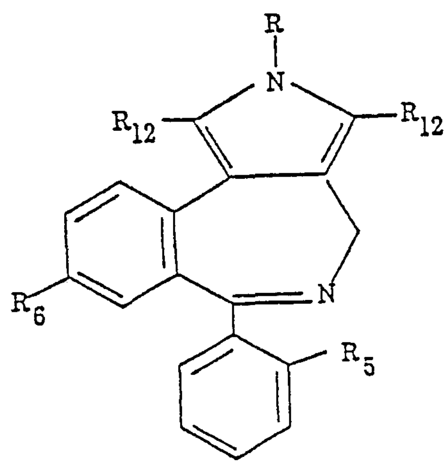

(III)

wherein $R_{12}$ signifies acetoxy and R, $R_5$ and $R_6$ have the significance given in claim 1, with a $(C_1\text{-}C_7)$-alkanol in the presence of a strong acid, or

f) for the manufacture of compounds of formula I defined in claim 1 in which $R_1$ or $R_3$ signifies hydrogen or $(C_1\text{-}C_7)$-alkyl and the remaining substituents have the significance given in claim 1, desoxidizing an N-oxide of the formula

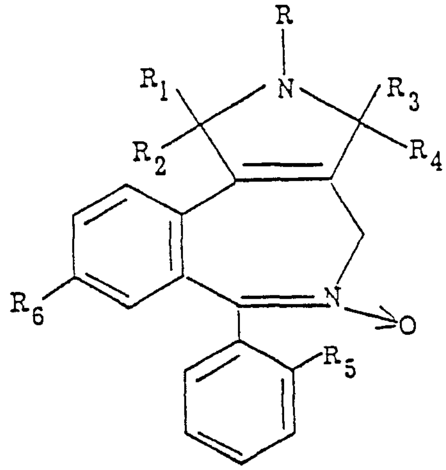

(IA)

wherein R, $R_1$, $R_2$, $R_3$, $R_4$, $R_5$ and $R_6$ have the significance given in claim 1, and, if desired,

g) converting a compound of formula I into a pharmaceutically acceptable salt or converting a pharmaceutically acceptable salt of a compound of formula I into another pharmaceutically acceptable salt.

21. A medicament containing a compound in accordance with any one of claims 1-17 and a therapeutically inert excipient.

22. A sedative and anxiolytic medicament containing a compound in accordance with any one of claims 1-17 and a therapeutically inert excipient.

**Claims** (for the Contracting State AT)

1. A process for the manufacture of pyrrolo[3,4-d]-[2]benzazepine derivatives of the general formula

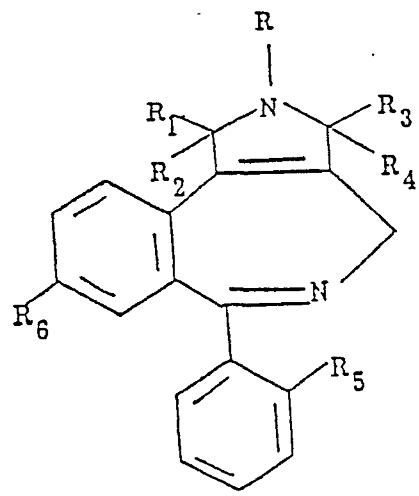

(I)

wherein $R_1$ and $R_3$ signify hydrogen, $(C_1$-$C_7)$-alkyl, hydroxy, $(C_1$-$C_7)$-alkoxy or the group —OCOR', R' signifies hydrogen, $(C_1$-$C_6)$-alkyl or phenyl and $R_2$ and $R_4$ signify hydrogen or $R_1$ and $R_2$ and/or $R_3$ and $R_4$ together signify oxo groups, with the proviso that at least one oxo group is present, R signifies hydrogen, $(C_1$-$C_7)$-alkyl, hydroxy-$(C_2$-$C_7)$-alkyl, amino-$(C_2$-$C_7)$-alkyl, mono- or di-$(C_1$-$C_7)$-alkylamino-$(C_2$-$C_7)$-alkyl or the group -$(C_1$-$C_6)$-alkyl-$COR^{21}$, $R^{21}$ signifies hydroxy, $(C_1$-$C_7)$-alkoxy, amino or mono- or di-$(C_1$-$C_7)$-alkylamino, $R_5$ signifies halogen with an atomic number not exceeding 35 or hydrogen and $R_6$ signifies halogen with an atomic number not exceeding 35, with the proviso that R signifies $(C_1$-$C_7)$-alkyl or hydrogen when $R_1$ or $R_3$ signifies hydroxy, $(C_1$-$C_7)$-alkoxy or —OCOR', of N-oxides and of pharmaceutically acceptable salts thereof, characterized by

a) for the manufacture of compounds of formula I above in which $R_1$ or $R_3$ signifies hydrogen or $(C_1$-$C_7)$-alkyl and the remaining symbols have the above significance and of N-oxides thereof, oxidizing a compound of the general formula

(See formula page 30)

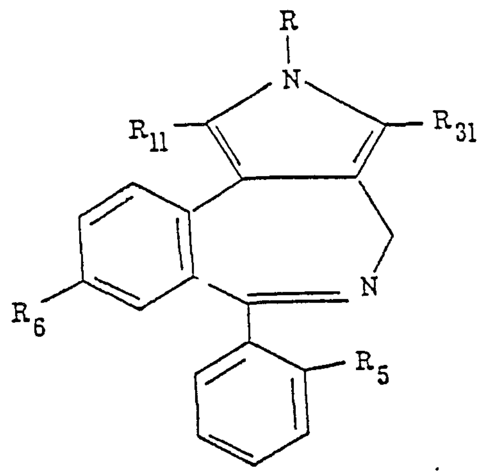

(II)

wherein one of $R_{11}$ and $R_{31}$ signifies hydrogen and the other signifies hydrogen or $(C_1$-$C_7)$-alkyl and R, $R_5$ and $R_6$ have the above significance, with a peracid, or

b) for the manufacture of compounds of formula I above in which $R_1$ or/$R_3$ signifies hydroxy and the remaining substituents have the above significance or $R_1$ and $R_2$ and $R_3$ and $R_4$ in each case together signify oxo groups and the remaining symbols have the above significance, oxidizing a compound of formula I in which $R_1$ or $R_3$ signifies hydrogen and the remaining substituents have the above significance or a compound of formula II above in which $R_{11}$ and $R_{31}$ signify hydrogen with a lead (IV) carboxylate in the presence of a strong acid, or

c) for the manufacture of compounds of formula I above in which $R_1$ or $R_3$ signifies acetoxy and the remaining substituents have the above significance, treating a compound of formula I in which $R_1$ or $R_3$ signifies hydrogen and the remaining substituents have the above significance or a compound of formula II above in which $R_{11}$ and $R_{31}$ signify hydrogen with lead tetraacetate in acetic acid, or

d) for the manufacture of compounds of formula I above in which $R_1$ or $R_3$ signifies —OCOR′ and the remaining substituents have the above significance, treating a compound of formula I in which $R_1$ or $R_3$ signifies hydroxy and the remaining substituents have the above significance with a carboxylic acid anhydride in the presence of a base, or

e) for the manufacture of compounds of formula I above in which $R_1$ or $R_3$ signifies $(C_1$-$C_7)$-alkoxy and the remaining substituents have the above significance, treating a compound of formula I in which $R_1$ or $R_3$ signifies —OCOR′ or hydroxy and the remaining substituents have the above significance or a compound of the formula

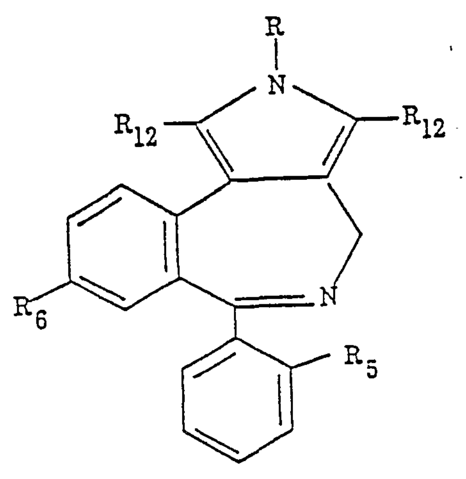

(III)

wherein $R_{12}$ signifies acetoxy and R, $R_5$ and $R_6$ have the above significance, with a $(C_1$-$C_7)$-alkanol in the presence of a strong acid, or

f) for the manufacture of compounds of formula I above in which $R_1$ or $R_3$ signifies hydrogen or $(C_1$-$C_7)$-alkyl and the remaining substituents have the above significance, desoxidizing an N-oxide of the general formula

(See formula page 31)

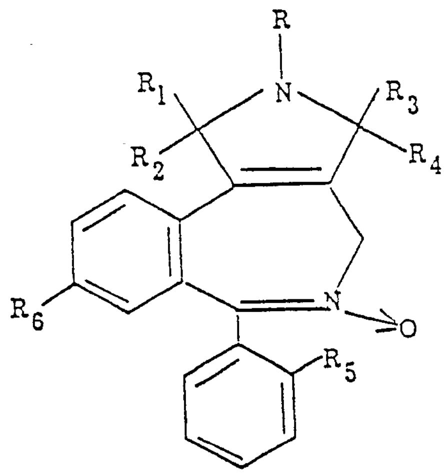

(IA)

wherein R, $R_1$, $R_2$, $R_3$, $R_4$, $R_5$ and $R_6$ have the above significance, and, if desired,

g) converting a compound of formula I into a pharmaceutically acceptable salt or converting a pharmaceutically acceptable salt of a compound of formula I into another pharmaceutically acceptable salt.

2. A process in accordance with claim 1, characterized in that a compound of formula I defined in claim 1 or a pharmaceutically acceptable acid addition salt thereof is manufactured.

3. A process in accordance with claim 1 or 2, characterized in that $R_3$ and $R_4$ together signify an oxo group.

4. A process in accordance with any one of claims 1-3, characterized in that $R_2$ signifies hydrogen.

5. A process in accordance with any one of claims 1-4, characterized in that $R_1$ signifies hydrogen or $(C_1$-$C_7)$-alkyl.

6. A process in accordance with claim 5, characterized in that $R_1$ signifies hydrogen or methyl.

7. A process in accordance with any one of claims 1-6, characterized in that R signifies hydrogen or $(C_1$-$C_7)$-alkyl.

8. A process in accordance with claim 7, characterized in that R signifies hydrogen or methyl.

9. A process in accordance with any one of claims 1-8, characterized in that $R_5$ signifies halogen with an atomic number not exceeding 35.

10. A process in accordance with claim 1, characterized in that 8-chloro-6-(2-chlorophenyl)-1,4-dihydropyrrolo[3,4-d][2]benzazepin-3(2H)-one is manufactured.

11. A process in accordance with claim 1, characterized in that 8-chloro-6-(2-chlorophenyl)-1,4-dihydro-1-methylpyrrolo[3,4-d][2]benzazepin-3(2H)-one is manufactured.

12. A process in accordance with claim 1, characterized in that 8-chloro-6-(2-chlorophenyl)-1,4-dihydro-2-methylpyrrolo[3,4-d][2]benzazepin-3(2H)-one is manufactured.

13. A process in accordance with claim 1, characterized in that 8-chloro-6-(2-fluorophenyl)-1,4-dihydropyrrolo[3,4-d][2]benzazepin-3(2H)-one is manufactured.

14. A process in accordance with claim 1, characterized in that 8-chloro-1,4-dihydro-6-phenylpyrrolo[3,4-d][2]benzazepin-3(2H)-one is manufactured.

15. A process in accordance with claim 1, characterized in that 8-chloro-6-(2-chlorophenyl)-1,4-dihydro-1,2-dimethylpyrrolo[3,4-d][2]benzazepin-3(2H)-one is manufactured.

16. A process in accordance with claim 1, characterized in that 8-chloro-6-(2-chlorophenyl)-1,4-dihydro-2-(2-hydroxyethyl)-1-methylpyrrolo[3,4-d][2]benzazepin-3(2H)-one is manufactured.

**Revendications** (pour les Etats contractants : BE, CH, DE, FR, GB, IT, LI, LU, NL, SE)

1. Dérivés de la pyrrolo[3,4-d][2]benzazépine de formule générale

(Voir formule page 32)

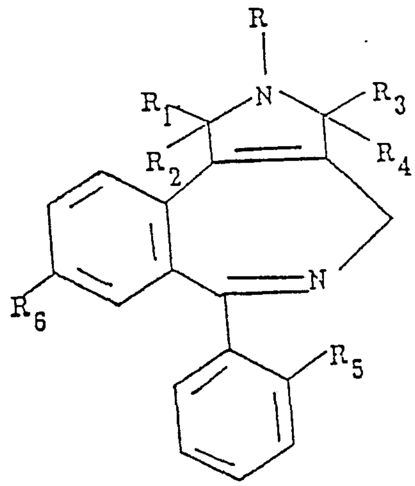

(I)

dans laquelle $R_1$ et $R_3$ représentent l'hydrogène, des groupes alkyle en C1-C7, hydroxy, alcoxy en C1-C7 ou le groupe —OCOR', R' représente l'hydrogène, un groupe alkyle en C1-C6 ou phényle et $R_2$ et $R_4$ représentent l'hydrogène, ou bien $R_1$ et $R_2$ et/ou $R_3$ et $R_4$ forment ensemble des groupes oxo, étant spécifié qu'il y a au moins un groupe oxo, R représente l'hydrogène, un groupe alkyle en C1-C7, hydroxyalkyle en C2-C7, aminoalkyle en C2-C7, mono- ou di-(alkyle en C1-C7)-aminoalkyle en C2-C7 ou le groupe -(alkyle en C1-C6)-$COR^{21}$, $R^{21}$ représente un groupe hydroxy, alcoxy en C1-C7, amino ou mono- ou di-(alkyle en C1-C7)-amino, $R_5$ représente un halogène de numéro atomique ne dépassant pas 35 ou l'hydrogène et $R_6$ représente un halogène de numéro atomique ne dépassant pas 35, étant spécifié que lorsque $R_1$ ou $R_3$ représente un groupe hydroxy, alcoxy en C1-C7 ou —OCOR', R représente un groupe alkyle en C1-C7, ou l'hydrogène, les N-oxydes et les sels acceptables pour l'usage pharmaceutique de ces composés.

2. Composés de formule générale I définie dans la revendication 1 et leurs sels acceptables pour l'usage pharmaceutique formés par addition avec des acides.

3. Composés selon la revendication 1 ou 2, dans lesquels $R_3$ et $R_4$ forment ensemble un groupe oxo.

4. Composés selon l'une des revendications 1 à 3, dans lesquels $R_2$ représente l'hydrogène.

5. Composés selon l'une des revendications 1 à 4, dans lesquels $R_1$ représente l'hydrogène ou un groupe alkyle en C1-C7.

6. Composés selon la revendication 5, dans lesquels $R_1$ représente l'hydrogène ou un groupe méthyle.

7. Composés selon l'une des revendications 1 à 6, dans lesquels R représente l'hydrogène ou un groupe alkyle en C1-C7.

8. Composés selon la revendication 7, dans lesquels R représente l'hydrogène ou un groupe méthyle.

9. Composés selon l'une des revendications 1 à 8, dans lesquels $R_5$ représente un halogène de numéro atomique ne dépassant pas 35.

10. La 8-chloro-6-(2-chlorophényl)-1,4-dihydropyrrolo[3,4-d][2]benzazépine-3(2H)-one.

11. La 8-chloro-6-(2-chlorophényl)-1,4-dihydro-1-méthyl-pyrrolo[3,4-d][2]benzazépine-3(2H)-one.

12. La 8-chloro-6-(2-chlorophényl)-1,4-dihydro-2-méthyl-pyrrolo[3,4-d][2]benzazépine-3(2H)-one.

13. La 8-chloro-6-(2-fluorophényl)-1,4-dihydropyrrolo[3,4-d][2]benzazépine-3(2H)-one.

14. La 8-chloro-1,4-dihydro-6-phénylpyrrolo[3,4-d][2]benzazépine-3(2H)-one.

15. La 8-chloro-6-(2-chlorophényl)-1,4-dihydro-1,2-diméthylpyrrolo[3,4-d][2]benzazépine-3(2H)-one.

16. La 8-chloro-6-(2-chlorophényl)-1,4-dihydro-2-(2-hydroxyéthyl)-1-méthylpyrrolo[3,4-d][2]benzazépine-3(2H)-one.

17. La 8-chloro-6-(2-fluorophényl)-3,4-dihydropyrrolo[3,4-d][2]benzazépine-1(2H)-one,
la 8-chloro-6-(2-chlorophényl)3,4-dihydropyrrolo[3,4-d][2]benzazépine-1(2H)-one,
la 8-chloro-3,4-dihydro-6-phénylpyrrolo[3,4-d][2]benzazépine-1(2H)-one,
la 8-chloro-6-(2-chlorophényl)-3,4-dihydro-2-méthylpyrrolo[3,4-d][2]benzazépine-1(2H)-one,
la 8-chloro-6-(2-chlorophényl)-1,4-dihydro-1-hydroxypyrrolo[3,4-d][2]benzazépine-3(2H)-one,
la 8-chloro-6-(2-chlorophényl)-3,4-dihydro-3-hydroxypyrrolo[3,4-d][2]benzazépine-1(2H)-one,
la 8-chloro-6-(2-chlorophényl)-pyrrolo[3,4-d][2]benzazépine-1,3(2H,4H)-dione,
le 5-oxyde de la 8-chloro-6-(2-chlorophényl)-1,4-dihydropyrrolo[3,4-d][2]benzazépine-3(2H)-one,
le 5-oxyde de la 8-chloro-6-(2-chlorophényl)-3,4-dihydropyrrolo[3,4-d][2]benzazépine-1(2H)-one,
la 8-chloro-6-(2-chlorophényl)-1-éthyl-1,4-dihydropyrrolo[3,4-d][2]benzazépine-3(2H)-one,
le 8-chloro-6-(2-chlorophényl)-1,4-dihydro-1-méthyl-3-oxo-2H-pyrrolo[3,4-d][2]benzazépine-2-acétate de méthyle, et
le 8-chloro-6-(2-chlorophényl)-1,4-dihydro-1-méthyl-3-oxo-2H-pyrrolo[3,4-d][2]benzazépine-2-acétamide.

18. Composés selon l'une des revendications 1 à 17, pour l'utilisation en tant que substances actives thérapeutiques.

19. Composés selon l'une des revendications 1 à 17, pour l'utilisation en tant que substances actives sédatives et anxiolytiques.

20. Procédé de préparation des composés selon l'une des revendications 1 à 17, caractérisé en ce que :

a) pour la préparation des composés de formule générale I définie dans la revendication 1 dans laquelle $R_1$ ou $R_3$ représente l'hydrogène ou un groupe alkyle en C1-C7, et les autres symboles ont les significations indiquées dans la revendication 1, et de leurs N-oxydes, on oxyde à l'aide d'un peracide un composé de formule générale

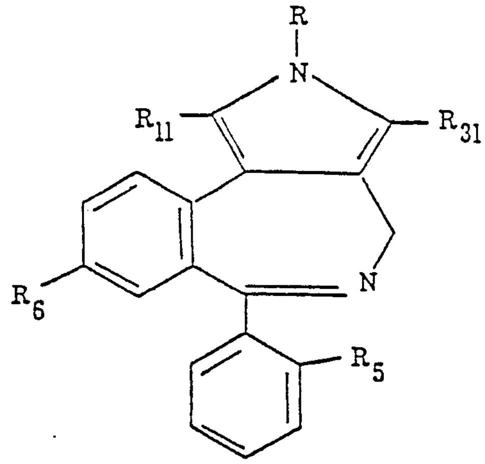

(II)

dans laquelle l'un des symboles $R_{11}$ et $R_{31}$ représentent l'hydrogène et l'autre l'hydrogène ou un groupe alkyle en C1-C7 et R, $R_5$ et $R_6$ ont les significations indiquées dans la revendication 1, ou bien

b) pour préparer les composés de formule générale I définie dans la revendication 1, dans laquelle $R_1$ ou $R_3$ représente un groupe hydroxy et les autres symboles ont les significations indiquées dans la revendication 1, ou bien $R_1$ et $R_2$ et $R_3$ et $R_4$ forment ensemble respectivement des groupes oxo, les autres symboles ayant les significations indiquées dans la revendication 1, on oxyde à l'aide d'un carboxylate de plomb-(IV), en présence d'un acide fort, un composé de formule I définie dans la revendication 1 dans laquelle $R_1$ ou $R_3$ représente l'hydrogène et les autres symboles ont les significations indiquées dans la revendication 1, ou un composé de formule II ci-dessus dans laquelle $R_{11}$ et $R_{31}$ représentent l'hydrogène, ou bien

c) pour préparer les composés de formule I définie dans la revendication 1, dans laquelle $R_1$ ou $R_3$ représente un groupe acétoxy et les autres symboles ont les significations indiquées dans la revendication 1, on traite par le tétracétate de plomb dans l'acide acétique un composé de formule I définie dans la revendication 1 dans laquelle $R_1$ ou $R_3$ représente l'hydrogène et les autres symboles ont les significations indiquées dans la revendication 1, ou bien un composé de formule II ci-dessus dans laquelle $R_{11}$ et $R_{31}$ représentent l'hydrogène, ou bien

d) pour préparer les composés de formule I définie dans la revendication 1 dans laquelle $R_1$ ou $R_3$ représente un groupe —OCOR' et les autres symboles ont les significations indiquées dans la revendication 1, on traite par un anhydride d'acide carboxylique en présence d'une base un composé de formule I définie dans la revendication 1 dans laquelle $R_1$ ou $R_3$ représente un groupe hydroxy et les autres symboles ont les significations indiquées dans la revendication 1, ou bien

e) pour préparer les composés de formule I définie dans la revendication 1 dans laquelle $R_1$ ou $R_3$ représente un groupe alcoxy en C1-C7 et les autres symboles ont les significations indiquées dans la revendication 1, on traite par un alcanol en C1-C7 en présence d'un acide fort un composé de formule I définie dans la revendication 1 dans laquelle $R_1$ ou $R_3$ représente un groupe —OCOR' ou hydroxy et les autres symboles ont les significations indiquées dans la revendication 1, ou bien un composé de formule

(Voir formule page 34)

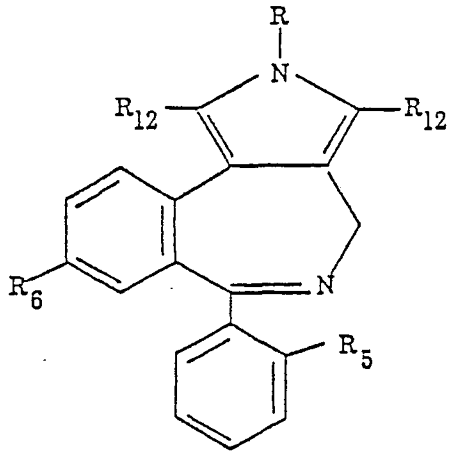

(III)

dans laquelle $R_{12}$ représente un groupe acétoxy et R, $R_5$ et $R_6$ ont les significations indiquées dans la revendication 1, ou bien

f) pour préparer les composés de formule I définie dans la revendication 1 dans laquelle $R_1$ ou $R_3$ représente l'hydrogène ou un groupe alkyle en C1-C7 et les autres symboles ont les significations indiquées dans la revendication 1, on désoxyde un N-oxyde de formule

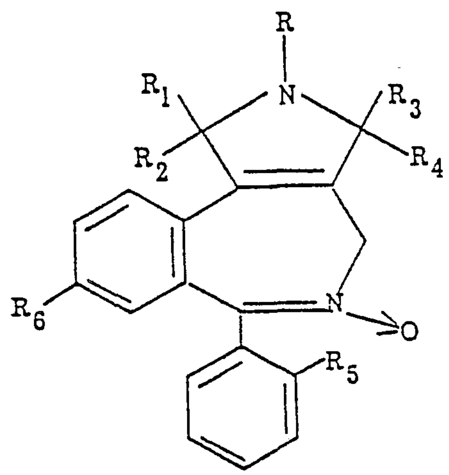

(IA)

dans laquelle R, $R_1$, $R_2$, $R_3$, $R_4$, $R_5$ et $R_6$ ont les significations indiquées dans la revendication 1, et si on le désire,

g) on convertit un composé de formule I en un sel acceptable pour l'usage pharmaceutique ou un sel acceptable pour l'usage pharmaceutique d'un composé de formule I en un autre sel acceptable pour l'usage pharmaceutique.

21. Médicament contenant un composé selon l'une des revendications 1 à 17 et un excipient inerte du point de vue thérapeutique.

22. Agent sédatif et anxiolytique contenant un composé selon l'une des revendications 1 à 17 et un excipient inerte du point de vue thérapeutique.

**Revendications** (pour l'Etat contractant AT)

1. Procédé de préparation de dérivés de la pyrrolo[3,4-d][2]benzazépine répondant à la formule générale I

(Voir formule page 35)

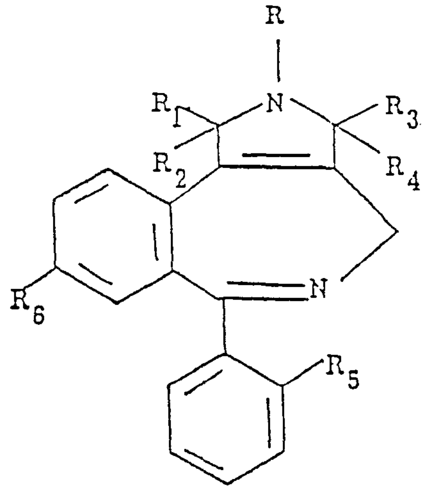

(I)

dans laquelle $R_1$ et $R_3$ représentent l'hydrogène, des groupes alkyle en C1-C7, hydroxy, alcoxy en C1-C7 ou le groupe —OCOR', R' représente l'hydrogène, un groupe alkyle en C1-C6 ou phényle et $R_2$ et $R_4$ représentent l'hydrogène ou bien $R_1$ et $R_2$ et/ou $R_3$ et $R_4$ forment ensemble des groupes oxo, étant spécifié qu'il y a au moins un groupe oxo, R représente l'hydrogène, un groupe alkyle en C1-C7, hydroxyalkyle en C2-C7, aminoalkyle en C2-C7 ou mono- ou di-(alkyle en C1-C7)-amino-alkyle en C2-C7 ou le groupe -(alkyle en C1-C6)-$COR^{21}$, $R^{21}$ représente un groupe hydroxy, alcoxy en C1-C7, amino ou mono- ou di-(alkyle en C1-C7)-amino, $R_5$ représente un halogène de numéro atomique ne dépassant pas 35 ou l'hydrogène et $R_6$ représente un halogène de numéro atomique ne dépassant pas 35, étant spécifié que lorsque $R_1$ ou $R_3$ représente un groupe hydroxy, alcoxy en C1-C7 ou —OCOR', R représente un groupe alkyle en C1-C7 ou l'hydrogène, de leurs N-oxydes et des sels acceptables pour l'usage pharmaceutique de ces composés, caractérisé en ce que :

a) pour préparer les composés de formule I ci-dessus dans laquelle $R_1$ ou $R_3$ représente l'hydrogène ou un groupe alkyle en C1-C7 et les autres symboles ont les significations indiquées ci-dessus, et leurs N-oxydes, on oxyde à l'aide d'un peracide un composé de formule générale

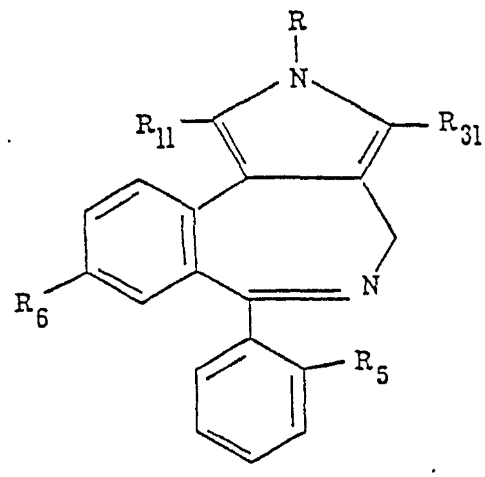

(II)

dans laquelle l'un des symboles $R_{11}$ et $R_{31}$ représente l'hydrogène et l'autre l'hydrogène ou un groupe alkyle en C1-C7, et R, $R_5$ et $R_6$ ont les significations indiquées ci-dessus, ou bien

b) pour préparer les composés de formule I ci-dessus dans laquelle $R_1$ ou $R_3$ représente un groupe hydroxy et les autres symboles ont les significations indiquées ci-dessus, ou bien dans laquelle $R_1$ et $R_2$ et $R_3$ et $R_4$ forment ensemble respectivement des groupes oxo, les autres symboles ayant les significations indiquées ci-dessus, on oxyde à l'aide d'un carboxylate de plomb-(IV) en présence d'un acide fort, un composé de formule I dans laquelle $R_1$ ou $R_3$ représente l'hydrogène et les autres symboles ont les significations indiquées ci-dessus, ou un composé de formule II ci-dessus dans laquelle $R_{11}$ et $R_{31}$ représentent l'hydrogène, ou bien

c) pour préparer les composés de formule I ci-dessus dans laquelle $R_1$ ou $R_3$ représente un groupe acétoxy et les autres symboles ont les significations indiquées ci-dessus, on traite par le tétracétate de plomb dans l'acide acétique un composé de formule I dans laquelle $R_1$ ou $R_3$ représente l'hydrogène et les autres symboles ont les significations indiquées ci-dessus, ou bien un composé de formule II ci-dessus dans laquelle $R_{11}$ et $R_{31}$ représentent l'hydrogène, ou bien

d) pour préparer les composés de formule I ci-dessus dans laquelle $R_1$ ou $R_3$ représente un groupe —OCOR' et les autres symboles ont les significations indiquées ci-dessus, on traite par un anhydride

d'acide carboxylique en présence d'une base, un composé de formule I dans laquelle $R_1$ ou $R_3$ représente un groupe hydroxy et les autres symboles ont les significations indiquées ci-dessus, ou bien

e) pour préparer les composés de formule I ci-dessus dans laquelle $R_1$ ou $R_3$ représente un groupe alcoxy en C1-C7 et les autres symboles ont les significations indiquées ci-dessus, on traite par un alcanol en C1-C7 en présence d'un acide fort, un composé de formule I dans laquelle $R_1$ ou $R_3$ représente un groupe —OCOR' ou un groupe hydroxy et les autres symboles ont les significations indiquées ci-dessus, ou bien un composé de formule

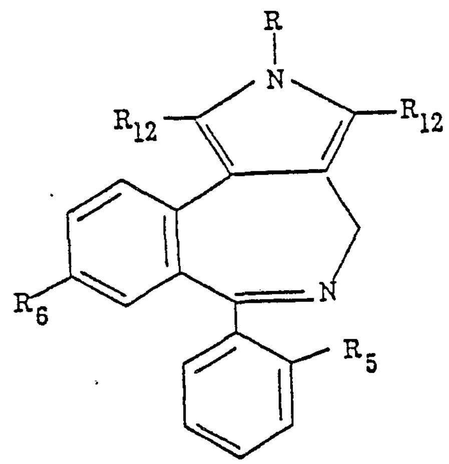

(III)

dans laquelle $R_{12}$ représente un groupe acétoxy et R, $R_5$ et $R_6$ ont les significations indiquées ci-dessus, ou bien

f) pour préparer les composés de formule I ci-dessus dans laquelle $R_1$ ou $R_3$ représente l'hydrogène ou un groupe alkyle en C1-C7 et les autres symboles ont les significations indiquées ci-dessus, on désoxyde un N-oxyde de formule générale

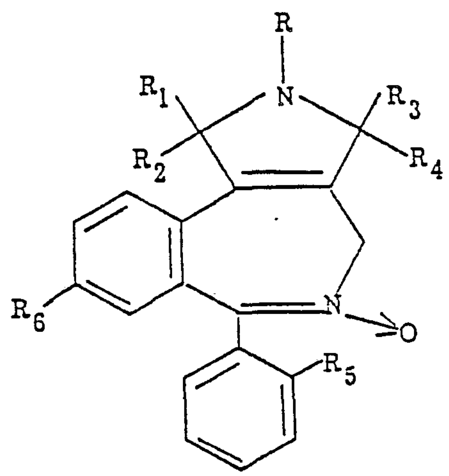

(IA)

dans laquelle R, $R_1$, $R_2$, $R_3$, $R_4$, $R_5$ et $R_6$ ont les significations indiquées ci-dessus, et si on le désire,

g) on convertit un composé de formule I en un sel acceptable pour l'usage pharmaceutique ou un sel acceptable pour l'usage pharmaceutique d'un composé de formule I en un autre sel acceptable pour l'usage pharmaceutique.

2. Procédé selon la revendication 1, caractérisé en ce que l'on prépare un composé de formule I définie dans la revendication 1 ou un sel d'un tel composé, acceptable pour l'usage pharmaceutique, formé par addition avec un acide.

3. Procédé selon la revendication 1 ou 2, caractérisé en ce que $R_3$ et $R_4$ forment ensemble un groupe oxo.

4. Procédé selon l'une des revendications 1 à 3, caractérisé en ce que $R_2$ représente l'hydrogène.

5. Procédé selon l'une des revendications 1 à 4, caractérisé en ce que $R_1$ représente l'hydrogène ou un groupe alkyle en C1-C7.

6. Procédé selon la revendication 5, caractérisé en ce que $R_1$ représente l'hydrogène ou un groupe méthyle.

7. Procédé selon l'une des revendications 1 à 6, caractérisé en ce que R représente l'hydrogène ou un groupe alkyle en C1-C7.

8. Procédé selon la revendication 7, caractérisé en ce que R représente l'hydrogène ou un groupe méthyle.

9. Procédé selon l'une des revendications 1 à 8, caractérisé en ce que $R_5$ représente un halogène de numéro atomique ne dépassant pas 35.

10. Procédé selon la revendication 1, caractérisé en ce que l'on prépare la 8-chloro-6-(2-chlorophényl)-1,4-dihydropyrrolo[3,4-d][2]benzazépine-3(2H)-one.

11. Procédé selon la revendication 1, caractérisé en ce que l'on prépare la 8-chloro-6-(2-chlorophényl)-1,4-dihydro-1-méthylpyrrolo[3,4-d][2]benzazépine-3(2H)-one.

12. Procédé selon la revendication 1, caractérisé en ce que l'on prépare la 8-chloro-6-(2-chlorophényl)-1,4-dihydro-2-méthylpyrrolo[3,4-d][2]benzazépine-3(2H)-one.

13. Procédé selon la revendication 1, caractérisé en ce que l'on prépare la 8-chloro-6-(2-fluorophényl)-1,4-dihydropyrrolo[3,4-d][2]benzazépine-3(2H)-one.

14. Procédé selon la revendication 1, caractérisé en ce que l'on prépare la 8-chloro-1,4-dihydro-6-phénylpyrrolo-[3,4-d][2]benzazépine-3(2H)-one.

15. Procédé selon la revendication 1, caractérisé en ce que l'on prépare la 8-chloro-6-(2-chlorophényl)-1,4-dihydro-1,2-diméthylpyrrolo[3,4-d][2]benzazépine-3(2H)-one.

16. Procédé selon la revendication 1, caractérisé en ce que l'on prépare la 8-chloro-6-(2-chlorophényl)-1,4-dihydro-2-(2-hydroxyéthyl)-1-méthylpyrrolo[3,4-d][2]benzazépine-3(2H)-one.